# EUROPEAN PATENT SPECIFICATION

(11) **EP 3 127 564 B1**
(45) Date of publication and mention of the grant of the patent: **26.12.2018**
(21) Application number: 15773632.3
(22) Date of filing: 31.03.2015
(51) Int. Cl.: A61M 1/18, A61M 1/16, A61M 1/34, A61M 1/36

(54) **BLOOD PURIFICATION DEVICE AND PRIMING METHOD FOR BLOOD PURIFICATION DEVICE**
BLUTREINIGUNGSVORRICHTUNG UND VERFAHREN ZUM BEFÜLLEN FÜR BLUTREINIGUNGSVORRICHTUNG
DISPOSITIF DE PURIFICATION DU SANG ET PROCÉDÉ D'AMORÇAGE DE FLUIDE POUR UN DISPOSITIF DE PURIFICATION DU SANG

(30) Priority: 31.03.2014 JP 2014074060; 08.10.2014 JP 2014207456
(43) Date of publication of application: 08.02.2017
(73) Proprietor: Med-tech Inc., Tokyo 101-0065 (JP); Asahi Kasei Medical Co., Ltd., Chiyoda-ku Tokyo 101-8101 (JP)
(72) Inventor: KATO, Yoko, Tokyo 101-8101 (JP); ZHANG, Liang, Tokyo 101-8101 (JP); KAWASHIMA, Masato, Tokyo 101-8101 (JP)
(74) Representative: dompatent von Kreisler Selting Werner - Partnerschaft von Patent- und Rechtsanwälten mbB
(86) International application number: PCT/JP2015/060112
(87) International publication number: WO 2015/152236

(56) References cited:
- EP-A1- 2 586 474
- EP-A2- 1 170 023
- WO-A1-2008/125893
- JP-A- 2010 184 029
- JP-A- 2010 184 029
- JP-A- 2011 110 098
- JP-A- 2012 192 102

## Description

### Technical Field

The present invention relates to a blood purification device and a fluid replacement/priming method for a blood purification device.

### Background Art

A blood purification device for performing a blood purification treatment such as hemodialysis includes a blood purification unit having a blood purification membrane such as a hollow fiber membrane. The blood purification device includes a blood circuit that supplies blood of a patient to the blood purification unit and returning the blood from the blood purification unit to the patient by using a blood pump and a dialysate circuit that supplies dialysate to the blood purification unit and discharges the dialysate from the blood purification unit. In this blood purification device, while maintaining extracorporeal circulation of blood in the blood circuit, dialysate in the dialysate circuit is supplied to the blood purification unit, and in the blood purification unit, blood passes through a primary side of the blood purification membrane and the dialysate passes through a secondary side of the blood purification membrane so that unwanted components in the blood are taken into the dialysate through the blood purification membrane for blood purification.

In a blood purification device of this type, it is necessary to purify a blood circuit through which blood passes before a start of a blood purification treatment. In view of this, before the blood purification treatment, so-called priming, which is a process of supplying cleaning fluid to the blood circuit to clean the blood circuit. In this priming, dialysate has been used instead of saline as cleaning fluid, and fresh dialysate in the dialysate circuit is caused to pass from a secondary side (a dialysate side) and flow into a primary side (a blood side) through a blood purification membrane of a blood purification unit (reverse filtration) so that the blood circuit is cleaned with the dialysate.

In addition, in a case where a blood pressure of a patient rapidly decreases in a blood purification process, for example, fluid replacement is performed on the patient. For this fluid replacement, dialysate has been used instead of saline in recent years, and fresh dialysate in a dialysate circuit is supplied to a blood circuit to perform fluid replacement on the patient.

Specifically, Patent Document 1, for example, describes that dialysate in a dialysate circuit is supplied to a blood circuit through a blood purification membrane by using a driving force of a water removing pump for adjusting the amount of water removed from a patient with the dialysate circuit. Patent Document 2 describes that dialysate in a dialysate circuit is supplied to a blood circuit through a blood purification membrane by using a driving force of a dialysate supply pump of the dialysate circuit.

In addition, Patent Document 3, for example, describes that dialysate in a dialysate circuit is urgently supplied to a patient through a blood circuit by using a driving force of a fluid replacement pump.

### Citation List

### Patent Documents

Patent Document 1: JP2002-325837
Patent Document 2: JP2011-110098
Patent Document 3: JP2010-184029

### Summary

### Technical Problem

However, since the water removing pump needs to strictly control the water removal amount of a patient, such a water removing pump generally has a low flow rate and a high flow rate accuracy. Thus, as described above, if the water removing pump is used as a pump for supplying dialysate to a blood circuit in priming, a small amount of dialysate is supplied, resulting in an increase in time for priming. In addition, if the water removing pump is operated at a maximum flow rate in its performance, a load is applied to the pump so that the pump can degrade, resulting in the possibility of inappropriate adjustment of a water removal amount in a subsequent blood purification treatment.

On the other hand, since the dialysate supply pump described above is used for supplying a large amount of dialysate to a blood purification unit, the pump generally has a high flow rate and a low flow rate accuracy. Thus, if a dialysate supply pump is used as a pump for supplying dialysate to a blood circuit in priming, the high flow rate causes a load to be applied to a blood purification membrane to break the blood purification membrane, resulting in a failure in performing a subsequent blood purification treatment suitably.

The present application has been made in view of the foregoing problems and has an object of providing a blood purification device that can suitably performing priming by using a pump having a flow rate and a flow rate accuracy suitable for the priming, and a fluid replacement/priming method for such a blood purification device. Solution to Problem

To achieve the objects described above, the present invention provides
1. a blood purification device comprising:
   a blood purification unit including a blood purification membrane;
   a blood circuit that feeds blood in a blood collection part to a primary side of the blood purification membrane of the blood purification unit and returns the blood from the primary side of the blood purification membrane of the blood purification unit to a blood return part, by using a blood pump;
   a dialysate supply circuit that supplies dialysate to a secondary side of the blood purification membrane of the blood purification unit;
   a dialysate discharge circuit that discharges the dialysate from the secondary side of the blood purification membrane of the blood purification unit; and
   a fluid replacement/priming circuit configured to perform
      a fluid replacement process in which the dialysate in the dialysate supply circuit is supplied to the blood circuit by using a fluid replacement pump and
      a priming process in which while the blood collection part and the blood return part are connected to each other to form a loop shape of the blood circuit, the dialysate in the dialysate supply circuit is caused to flow out to the primary side through the blood purification membrane and the dialysate in the blood circuit including the primary side is discharged to the dialysate discharge circuit by using the fluid replacement pump, characterized in that
   the fluid replacement/priming circuit is connected to the blood circuit, and is configured to be selectively connectable to the dialysate supply circuit and the dialysate discharge circuit, and
   the fluid replacement/priming circuit includes a bypass circuit connecting the dialysate supply circuit to the dialysate discharge circuit, and a connection circuit connecting the blood circuit to an intermediate portion of the bypass circuit,
   the bypass circuit includes a first flow rate adjusting unit disposed between a connection point to the connection circuit and a connection point to the dialysate discharge circuit and configured to be freely opened and closed, and a second flow rate adjusting unit disposed between a connection point to the connection circuit and a connection point to the dialysate supply circuit and configured to be freely opened and closed, and
   the fluid replacement pump is rotatable forwardly and reversely and is disposed in the connection circuit.
2. The blood purification device according to item 1, wherein
   the fluid replacement/priming circuit is configured to perform a priming second process in which while the blood collection part and the blood return part are connected to each other to form a loop shape of the blood circuit, the dialysate in the dialysate supply circuit is caused to flow out to the primary side through the blood purification membrane and the dialysate in the blood circuit including the primary side is returned to the dialysate supply circuit by using the fluid replacement pump.
3. The blood purification device according to any one of items 1 or 2, wherein
   the blood circuit includes a blood collection circuit connecting the blood collection part to the blood purification unit and a blood return circuit connecting the blood purification unit to the blood return part, and
   the connection circuit is configured to be connectable to at least one of the blood collection circuit or the blood return circuit.
4. The blood purification device according to any one of items 1 to 3, further comprising a blood returning circuit connecting a portion of the blood circuit closer to the blood collection part than the blood pump to the connection circuit.
5. The blood purification device according to any one of items 1 to 4, further comprising:
   a pressure sensor configured to detect a pressure of at least one of the primary side or the secondary side of the blood purification membrane; and
   a control unit that adjusts a flow rate of the fluid replacement pump so that a pressure on the blood purification membrane is within a predetermined range, based on a result of detection by the pressure sensor.
6. The blood purification device according to any one of items 1 to 5, further comprising a control unit that adjusts a flow rate of the blood pump so that the flow rate of the blood pump is lower than a flow rate of the fluid replacement pump in the priming process.
7. The blood purification device according to any one of items 1 or 2, wherein
   the dialysate supply circuit includes a first channel having a plurality of filters and a second channel having one or more filters in a number smaller than the number of the filters in the first channel, and is configured to be switchable between the first channel and the second channel.
8. The blood purification device according to item 7, further comprising a switching unit that performs switching between the first channel and the second channel in such a manner that in the case of performing a backfiltration process in which a part or whole of the dialysate supplied to the secondary side of the blood purification membrane of the blood purification unit is supplied to the primary side through the blood purification membrane, the dialysate is supplied to the blood purification unit through the first channel, whereas in the case of not performing the backfiltration process, the dialysate is supplied to the blood purification unit through the second channel.
9. The blood purification device according to item 7 or 8, further comprising
   a fluid replacement circuit connecting a downstream side of the plurality of filters in the first channel to the blood circuit, wherein
   the dialysate supply circuit is configured to supply dialysate to the fluid replacement circuit through the first channel.
10. The blood purification device according to item 9, further comprising
   a connection circuit connecting the fluid replacement circuit to the dialysate discharge circuit, wherein
   the blood purification device is configured to be switchable between connection to the first channel of the fluid replacement circuit and connection to the dialysate discharge circuit.
11. The blood purification device according to any one of items 7 to 10, wherein the first channel and the second channel share one or more of the filters.
12. The blood purification device according to item 11, wherein
   the one or more filters shared by the first and second channels are disposed upstream of the other filters.
13. The blood purification device according to any one of items 7 to 12, wherein
   in a case where the flow rate of dialysate supplied to a most upstream filter is less than 700 mL/min, the dialysate is distributed to the first channel, whereas in a case where the flow rate is 700 mL/min or higher, the dialysate is distributed to the second channel.
14. A method for priming of the blood purification device (1) as defined in claim 1, the method comprising
   a step of, while connecting a blood collection part and a blood return part of a blood circuit to each other to form a loop shape of the blood circuit, supplying dialysate to a secondary side of a blood purification membrane of a blood purification unit through filters in a dialysate supply circuit, causing the dialysate to flow out to a primary side through the blood purification membrane, and discharging the dialysate in the blood circuit including the primary side to a dialysate discharge circuit, wherein the number of filters through which the dialysate passes in the dialysate supply circuit in the step is larger than the number of filters through which the dialysate passes in the dialysate supply circuit in a blood purification process.

### Advantageous Effects of Invention

According to the present invention, priming can be suitably performed by using a pump having a flow rate and a flow rate accuracy suitable for the priming.

### Brief Description of Drawings

Fig. 1 schematically illustrates a configuration of a blood purification device according to a first embodiment.
Fig. 2 illustrates a priming first process of the device illustrated in Fig. 1.
Fig. 3 illustrates the priming first process of the device illustrated in Fig. 1.
Fig. 4 illustrates a priming second process of the device illustrated in Fig. 1.
Fig. 5 illustrates a blood purification process of the device illustrated in Fig. 1.
Fig. 6 illustrates urgent fluid replacement of the device illustrated in Fig. 1.
Fig. 7 illustrates a configuration of a blood purification device including a blood return circuit.
Fig. 8 illustrates a configuration of a blood purification device according to a second embodiment.
Fig. 9 illustrates a priming first process of the device illustrated in Fig. 8.
Fig. 10 illustrates a priming second process of the device illustrated in Fig. 8.
Fig. 11 illustrates a blood purification process of the device illustrated in Fig. 8.
Fig. 12 illustrates urgent fluid replacement of the device illustrated in Fig. 8.
Fig. 13 illustrates a configuration of a blood purification device according to a third embodiment.
Fig. 14 illustrates a priming first process of the device illustrated in Fig. 13.
Fig. 15 illustrates a priming second process of the device illustrated in Fig. 13.
Fig. 16 illustrates a blood purification process of the device illustrated in Fig. 13.
Fig. 17 illustrates urgent fluid replacement of the device illustrated in Fig. 13.
Fig. 18 schematically illustrates a configuration of a blood purification device according to a fourth embodiment.
Fig. 19 illustrates a priming first process of the device illustrated in Fig. 18.
Fig. 20 illustrates a priming second process of the device illustrated in Fig. 18.
Fig. 21 illustrates a blood purification process of the device illustrated in Fig. 18.
Fig. 22 illustrates urgent fluid replacement of the device illustrated in Fig. 18.
Fig. 23 illustrates a configuration of a blood purification device according to a fifth embodiment.
Fig. 24 illustrates a priming first process of the device illustrated in Fig. 23.
Fig. 25 illustrates a priming second process of the device illustrated in Fig. 23.
Fig. 26 illustrates a blood purification process of the device illustrated in Fig. 23.
Fig. 27 illustrates urgent fluid replacement of the device illustrated in Fig. 23.
Fig. 28 illustrates a configuration of a blood purification device according to a sixth embodiment.
Fig. 29 illustrates a priming second process of the device illustrated in Fig. 28.
Fig. 30 illustrates a blood purification process of the device illustrated in Fig. 28.
Fig. 31 illustrates urgent fluid replacement of the device illustrated in Fig. 28.
Fig. 32 schematically illustrates a configuration of a blood purification device.
Fig. 33 illustrates a state of the blood purification device in priming.
Fig. 34 illustrates a state of the blood purification device in a blood purification process.
Fig. 35 illustrates a state of the blood purification device in another blood purification process.
Fig. 36 illustrates another configuration of a second dialysate supply circuit.

### Description of Embodiments

Examples of preferred embodiments of the present invention will be described hereinafter with reference to the drawings. Positional relationships in vertical and horizontal directions in the drawings are based on the illustrated positional relationships, unless otherwise specified. Dimensional proportions in the drawings are not limited to illustrated proportions. The following embodiments are examples for describing the present invention.

### First Embodiment

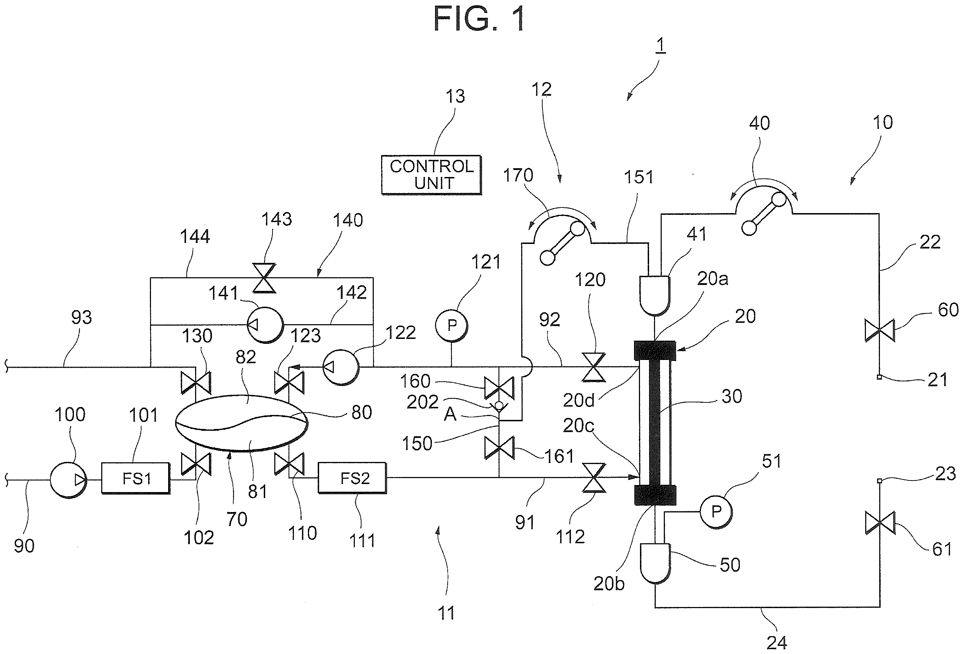

Fig. 1 schematically illustrates a configuration of a blood purification device 1 according to the present embodiment. The blood purification device 1 includes, for example, a blood circuit 10, a dialysate circuit 11, a fluid replacement/priming circuit 12, and a control unit 13.

The blood circuit 10 includes, for example, a blood collection circuit 22 connecting a blood collection part 21 and a blood purification unit 20 to each other and a blood return circuit 24 connecting the blood purification unit 20 and a blood return part 23 to each other.

The blood purification unit 20 includes a blood purification membrane 30 of, for example, a hollow fiber membrane in a cylindrical module. The blood purification unit 20 includes an inlet part 20a and an outlet part 20b communicating with a primary side (a blood side) of the blood purification membrane 30, and also includes an inlet part 20c and an outlet part 20d communicating with a secondary side (a dialysate side) of the blood purification membrane 30.

The blood collection circuit 22 is constituted by a soft tube and is connected to the inlet part 20a of the blood purification unit 20. The blood collection circuit 22 includes a blood pump 40 and a drip chamber 41. The blood pump 40 can be, for example, a pump that is rotatable forwardly and reversely. The blood pump 40 is, for example, a tubing pump that has a flow rate ranging from about 0 to 600 mL/min and can supply about 10 mL in one turn.

The blood return circuit 24 is constituted by, for example, a soft tube and is connected to the outlet part 20b of the blood purification unit 20. The blood return circuit 24 includes, for example, a drip chamber 50 and a pressure sensor 51.

The blood collection circuit 22 and the blood return circuit 24 include clamp valves 60 and 61, respectively, so that portions near the blood collection part 21 and the blood return part 23 can be opened and closed.

The dialysate circuit 11 includes, for example, a metering pump 70. The metering pump 70 includes a partition 80 that can be freely displaced in a body having a constant volume. The partition 80 defines a dialysate supply chamber 81 and a dialysate discharge chamber 82.

The dialysate circuit 11 includes a first dialysate supply circuit 90 connected from an unillustrated dialysate supply source to the dialysate supply chamber 81 of the metering pump 70 and a second dialysate supply circuit 91 connected from the dialysate supply chamber 81 of the metering pump 70 to the secondary-side inlet part 20c of the blood purification unit 20. The dialysate circuit 11 also includes a first dialysate discharge circuit 92 connected from the secondary-side outlet part 20d at the secondary side of the blood purification unit 20 to the dialysate discharge chamber 82 of the metering pump 70 and a second dialysate discharge circuit 93 connected from the dialysate discharge chamber 82 of the metering pump 70 to the outside of the device. These first dialysate supply circuit 90, the second dialysate supply circuit 91, the first dialysate discharge circuit 92, and the second dialysate discharge circuit 93 are constituted by, for example, soft tubes.

In the first dialysate supply circuit 90, a dialysate supply pump 100, a flow rate sensor 101, and an opening/closing valve 102 are arranged in this order from the upstream side, for example. The dialysate supply pump 100 can be, for example, a gear pump having a flow rage ranging from about 0 to 1500 mL/min and varying in accordance with a discharge pressure.

In the second dialysate supply circuit 91, an opening/closing valve 110, a flow rate sensor 111, and an opening/closing valve 112 are arranged in this order from the upstream side, for example.

In the first dialysate discharge circuit 92, an opening/closing valve 120, a pressure sensor 121, a discharge pump 122, and an opening/closing valve 123 are arranged in this order from the upstream side, for example.

In the second dialysate discharge circuit 93, an opening/closing valve 130, for example, is provided.

The dialysate circuit 11 includes a water removing circuit 140 directly connecting the first dialysate discharge circuit 92 to the second dialysate discharge circuit 93 with no metering pump 70 interposed therebetween. The water removing circuit 140 includes, for example, a first circuit 142 having a water removing pump 141 and a second circuit 144 having an opening/closing valve 143. The first circuit 142 and the second circuit 144 are connected to each other in parallel. The water removing pump 141 can be, for example, a piston pump having a flow rate ranging from about 0 to 90 mL/min and supplying about 0.5 to 1.0 mL in one stroke.

The fluid replacement/priming circuit 12 includes, a bypass circuit 150 connecting the second dialysate supply circuit 91 to the first dialysate discharge circuit 92 and a connection circuit 151 connected from the drip chamber 41 of the blood collection circuit 22 to an intermediate portion of the bypass circuit 150. The bypass circuit 150 and the connection circuit 151 are constituted by, for example, soft tubes.

The bypass circuit 150 includes an opening/closing valve (first flow rate adjusting unit) 160 and an opening/closing valve (second flow rate adjusting unit) 161 at both sides sandwiching a connection point A to the connection circuit 151. The flow rate adjusting units herein can be needle valves or rotary solenoids as well as opening/closing valves. Between the connection point A to the bypass circuit 150 and the opening/closing valve opening/closing valve 160, a check valve 202 for preventing a backflow from the first dialysate discharge circuit 92 to the second dialysate supply circuit 91 is disposed.

The connection circuit 151 includes a fluid replacement pump 170 that is rotatable forwardly and reversely. The fluid replacement pump 170 can be, for example, a tubing pump that has a flow rate ranging from about 0 to 600 mL/min and can supply about 10 mL in one turn.

The control unit 13 is, for example, a computer for controlling an entire operation of the blood purification device 1, and can control operations of, for example, the pumps 40, 100, 122, 141, and 170 and the valves 102, 110, 112, 120, 123, 130, 143, 160, and 161 and causes the blood purification device 1 to operate and perform priming and a blood purification process described later.

Priming using the blood purification device 1 with the foregoing configuration will be described.

When the blood purification unit 20 is placed in the blood purification device 1 and the circuits are connected, for example, priming is first performed. In the priming, first, dialysate is supplied from the secondary-side inlet part 20c of the blood purification membrane 30 of the blood circuit 10 so that fluid replacement is performed.

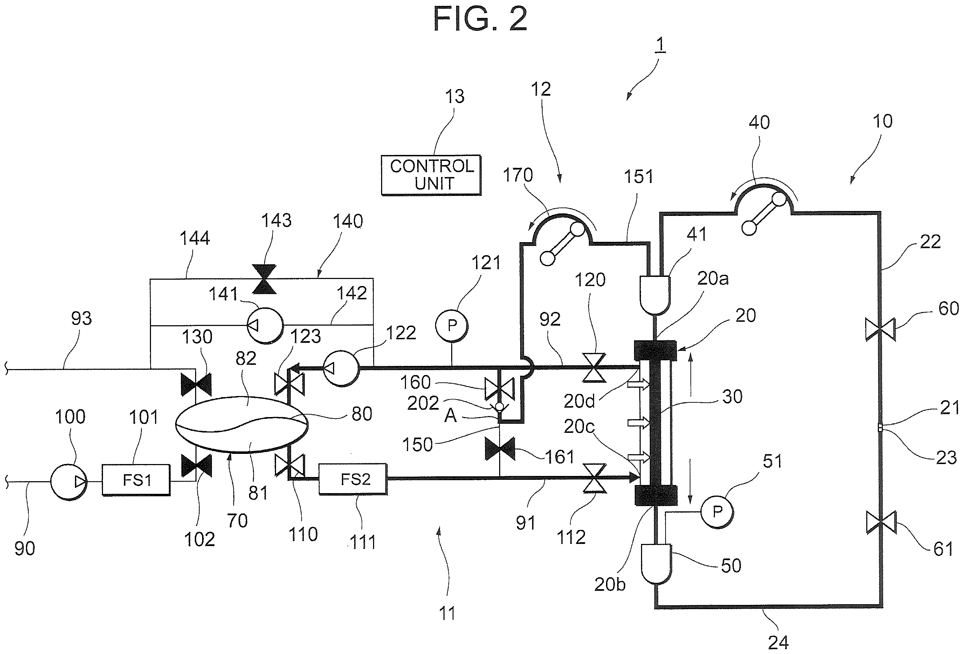

Next, as illustrated in Fig. 2, for example, in the dialysate circuit 11, the partition 80 of the metering pump 70 is driven by driving the discharge pump 122 so that dialysate is supplied from the dialysate supply chamber 81 of the metering pump 70 to the secondary side of the blood purification membrane 30 of the blood purification unit 20 through the second dialysate supply circuit 91 and then is discharged from the secondary side of the blood purification unit 20 to the dialysate discharge chamber 82 of the metering pump 70 through the first dialysate discharge circuit 92. When the dialysate is completely consumed in the dialysate supply chamber 81, dialysate is supplied through the first dialysate supply circuit 90.

In the fluid replacement/priming circuit 12, the opening/closing valve 160 is opened, the opening/closing valve 161 is closed, and the fluid replacement pump 170 is rotated reversely toward the dialysate circuit 11 (in the direction opposite to a fluid replacement direction). In the blood circuit 10, while the blood collection part 21 and the blood return part 23 are connected to each other, the blood pump 40 is rotated forwardly (in the same direction as a blood treatment). At this time, the control unit 13 adjusts the flow rate of the blood pump 40 so that the flow rate of the blood pump 40 is lower than the flow rate of the fluid replacement pump 170. In this manner, the dialysate at the secondary side of the blood purification membrane 30 of the blood purification unit 20 flows into the primary side through the blood purification membrane 30, and flows out into the blood collection circuit 22 and the blood return circuit 24 from both the inlet part 20a and the outlet part 20b of the blood purification unit 20. The dialysate is discharged from the drip chamber 41 of the blood collection circuit 22 to the first dialysate discharge circuit 92 through the connection circuit 151 and the bypass circuit 150 of the fluid replacement/priming circuit 12 (priming first process). As a result, fluid in the blood circuit 10 and the fluid replacement/priming circuit 12 is replaced and cleaned with fresh dialysate, and bubbles in the blood circuit 10 and the fluid replacement/priming circuit 12 are removed.

In the priming first process, the removed bubbles are compressed by the discharge pump 122 through the first dialysate discharge circuit 92 and discharged to the dialysate discharge chamber 82 of the metering pump 70 so that a supply/discharge balance of the dialysate in the metering pump 70 (where the amount of dialysate discharged from the dialysate supply chamber 81 needs to be equal to the amount of dialysate flowing into the dialysate discharge chamber 82) is disturbed and the dialysate circuit 11 comes to have a negative pressure in some cases.

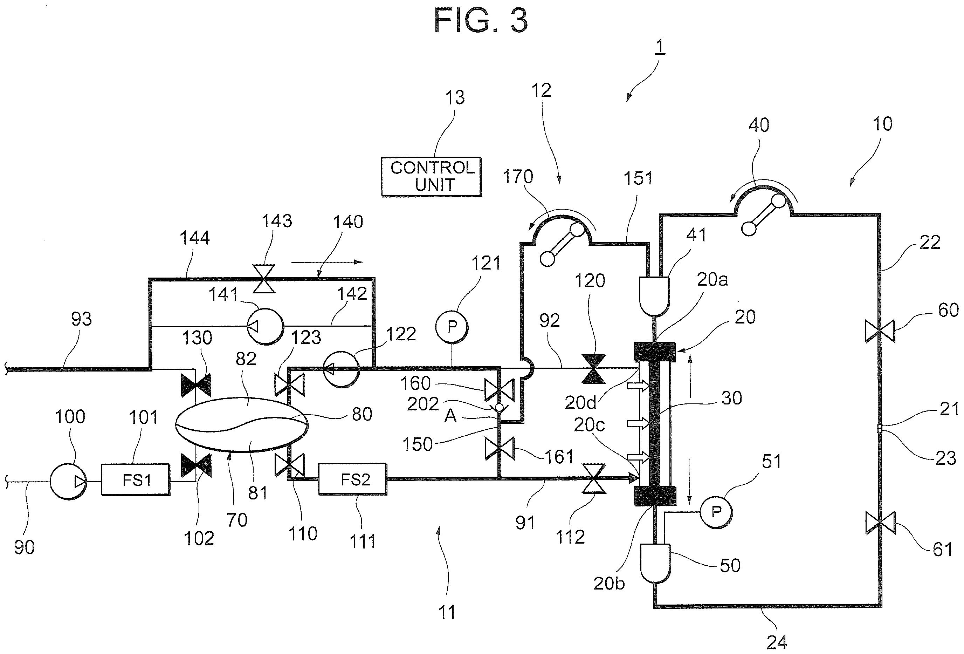

At this time, as illustrated in Fig. 3, for example, while the opening/closing valve 120 is closed and the opening/closing valve 161 is open, the opening/closing valve 143 of the water removing circuit 140 is opened, the dialysate in an amount corresponding to the volume of the compressed bubbles reversely flows in the second circuit 144 to be supplied to the dialysate discharge chamber 82. In this manner, a supply/discharge balance of the dialysate in the metering pump 70 in the priming first process (where the amount of dialysate discharged from the dialysate supply chamber 81 needs to be equal to the amount of dialysate flowing into the dialysate discharge chamber 82) can be obtained. At this time, the flow rate in the second dialysate supply circuit 91 is kept at a predetermined value or more so that a detection sensitivity of a flow rate sensor such as the flow rate sensor 111 can be maintained. In addition, the check valve 202 can prevent the dialysate in the first dialysate discharge circuit 92 from flowing reversely in the bypass circuit 150 and being supplied to the secondary side of the blood purification membrane 30 of the blood purification unit 20. Since the opening/closing valve 120 is closed, it is possible to prevent the dialysate in the first dialysate discharge circuit 92 from being supplied to the secondary side of the blood purification membrane 30 of the blood purification unit 20. This process may be performed at each priming first process or may be performed when necessary instead of being performed at each priming first process.

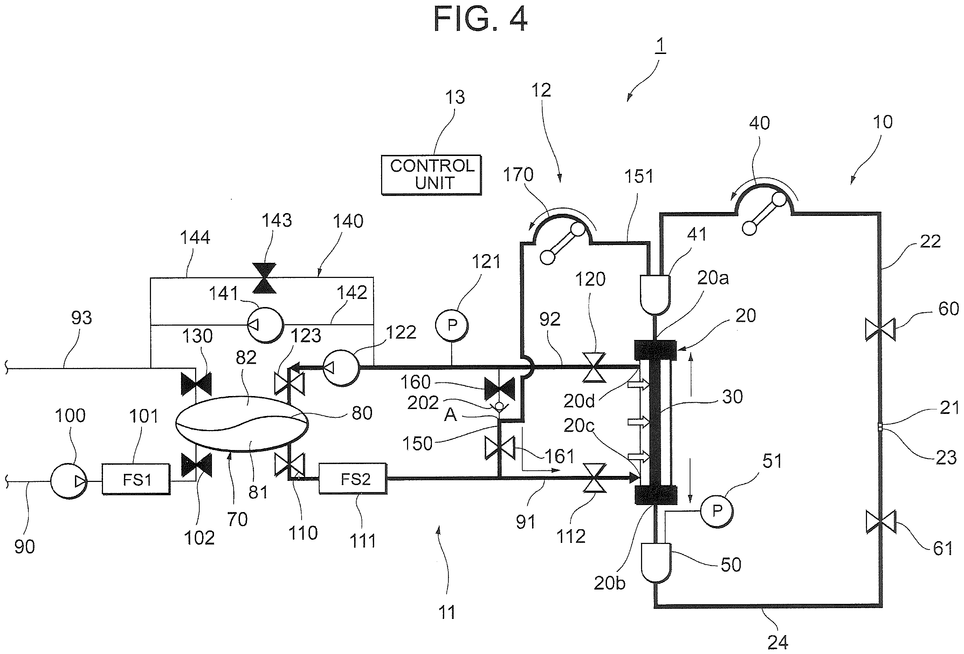

After the priming first process has been performed for a predetermined time, the opening/closing valve 160 is closed and the opening/closing valve 161 is opened, as illustrated in Fig. 4. In this manner, dialysate supplied to the bypass circuit 150 through the connection circuit 151 by using the fluid replacement pump 170 is returned to the second dialysate supply circuit 91, is supplied to the blood purification unit 20 again, and circulates therein (priming second process). In this priming second process, by using the fluid replacement pump 170 and the blood pump 40, dialysate in the second dialysate supply circuit 91 flows into the blood collection circuit 22 and the blood return circuit 24 of the blood circuit 10 through the blood purification membrane 30, is discharged from the drip chamber 41 to the connection circuit 151 through the blood circuit 10, is returned to the second dialysate supply circuit 91 through the connection circuit 151 and the bypass circuit 150, and is partially discharged to the first dialysate discharge circuit 92 through the secondary side of the blood purification membrane 30. In this manner, cleaning of the blood circuit 10 and the fluid replacement/priming circuit 12 continues.

In these priming processes, a flushing operation of opening and closing the clamp valves 60 and 61 may be performed to detach bubbles from inner walls of tubes of the blood circuit 10 and the blood purification membrane 30 and promote removal of bubbles, or the flow rate of the blood pump 40 may be changed to promote removal of bubbles. The flow rate of the blood pump 40 may be higher than the flow rate of the fluid replacement pump 170, or the blood pump 40 may be rotated reversely (in the direction opposite to a blood treatment).

In the priming processes, the pressure at the primary side of the blood purification membrane 30 may be monitored by the pressure sensor 51, for example. Based on a result of the pressure detection by the pressure sensor 51, the control unit 13, for example, may adjust the flow rate of the fluid replacement pump 170 so that a pressure on the blood purification membrane 30 is within a predetermined range. Specifically, the flow rate of the fluid replacement pump 170 may be feedback-controlled so as to prevent the pressure at the primary side of the blood purification membrane 30 from being lower than, for example, -300 mmHg. The pressure sensor 51 for detecting the pressure at the primary side of the blood purification membrane 30 is not necessarily disposed in the drip chamber 50, and may be disposed at another location in the blood circuit 10. Based on a result of detection of a pressure at the secondary side of the blood purification membrane 30, the flow rate of the fluid replacement pump 170 may be adjusted so that the pressure on the blood purification membrane 30 is within a predetermined range.

After the priming first process and the priming second process have been performed for predetermined times, priming is finished.

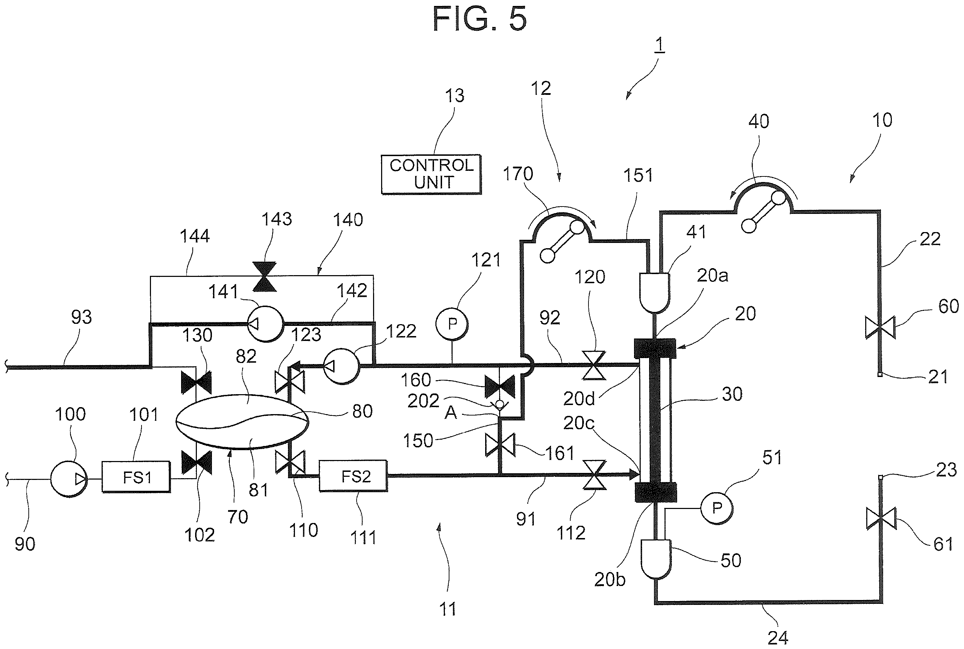

Then, a blood purification process, which does not form part of the invention, performed in the blood purification device 1 will be described. An unillustrated paracentesis needle is connected to the blood collection part 21 and the blood return part 23 and put into a patient. As illustrated in Fig. 5, in the blood circuit 10, blood of the patient is supplied to the primary side of the blood purification membrane 30 of the blood purification unit 20 through the blood collection circuit 22 by forward rotation (indicated by an arrow) of the blood pump 40, and the blood that has passed through the primary side of the blood purification membrane 30 is returned to the patient through the blood return circuit 24.

On the other hand, in the dialysate circuit 11, the partition 80 of the metering pump 70 is driven by the discharge pump 122 so that dialysate in the dialysate supply chamber 81 of the metering pump 70 is supplied to the secondary side of the blood purification membrane 30 of the blood purification unit 20 through the second dialysate supply circuit 91, and the dialysate that has passed through the secondary side of the blood purification membrane 30 is discharged to the dialysate discharge chamber 82 of the metering pump 70 through the first dialysate discharge circuit 92.

In the blood purification unit 20, blood flows into the primary side of the blood purification membrane 30 and dialysate flows into the secondary side so that waste products in the blood are discharged to the dialysate through the blood purification membrane 30 and, thereby, the blood is purified.

In the water removing circuit 140, the water removing pump 141 is driven so that dialysate in the first dialysate discharge circuit 92 is discharged to the second dialysate discharge circuit 93 through the water removing circuit 140 without passing through the metering pump 70. The amount of dialysate passing through the water removing circuit 140 at this time is adjusted in accordance with the amount of water removed from the blood of the patient.

In addition, in the fluid replacement/priming circuit 12, while the opening/closing valve 161 is open and the opening/closing valve 160 is closed, the fluid replacement pump 170 is rotated forwardly (in the direction indicated by an arrow) when necessary, and dialysate in the second dialysate supply circuit 91 is partially supplied to the blood circuit 10 through the bypass circuit 150 and the connection circuit 151. In this manner, fluid replacement is performed on the patient (fluid replacement process).

For a predetermined time, blood circulation in the blood circuit 10 and supply/discharge of dialysate in the dialysate circuit 11 are performed, and then the blood purification process is finished.

In the present embodiment, in the fluid replacement/priming circuit 12, the priming first process can be performed in such a manner that dialysate in the second dialysate supply circuit 91 flows out to the primary side through the blood purification membrane 30 by using the fluid replacement pump 170, and the dialysate in the blood circuit 10 including the primary side is discharged to the first dialysate discharge circuit 92 without passing through the blood purification membrane 30. Thus, priming can be suitably performed by using the fluid replacement pump 170 having a flow rate and a flow rate accuracy suitable for the priming.

After the priming first process, the priming second process can be performed in such a manner that dialysate in the second dialysate supply circuit 91 flows out to the primary side through the blood purification membrane 30 by using the fluid replacement pump 170, and the dialysate in the blood circuit 10 including the primary side is returned to the second dialysate supply circuit 91 without passing through the blood purification membrane 30 and is supplied to the blood purification unit 20 again. Thus, the amount of the dialysate used in priming can be reduced. Since bubbles cannot pass through the wet blood purification membrane 30, bubbles discharged from the blood circuit 10 to the fluid replacement/priming circuit 12 do not enter the blood circuit 10 through the blood purification membrane 30 again.

In addition, since the flow rate of the fluid replacement pump 170 can be adjusted so that a pressure on the blood purification membrane 30 is not higher than a predetermined threshold, it is possible to prevent damage of the blood purification membrane 30 under an excessive load.

Furthermore, since the blood pump 40 of the blood collection circuit 22 is rotated forwardly in priming and the flow rate of the blood pump 40 is lower than that of the fluid replacement pump 170, dialysate flowed into the primary side of the blood purification membrane 30 of the blood purification unit 20 flows out to both sides, that is, to the blood collection circuit 22 and the blood return circuit 24. In this manner, clean dialysate can always flow out from the blood purification membrane 30 to the outside, thereby ensuring cleaning of the blood purification membrane 30 and removal of bubbles.

Since the fluid replacement/priming circuit 12 is configured to be selectively connectable to the second dialysate supply circuit 91 and the first dialysate discharge circuit 92, the same circuit can be suitably switched between fluid replacement and priming.

Since the fluid replacement/priming circuit 12 includes the bypass circuit 150 and the connection circuit 151, the bypass circuit 150 includes the opening/closing valves 160 and 161, and the fluid replacement pump 170 can be rotated forwardly and reversely and is provided in the connection circuit 151, fluid replacement and priming can be suitably performed by using the fluid replacement pump 170.

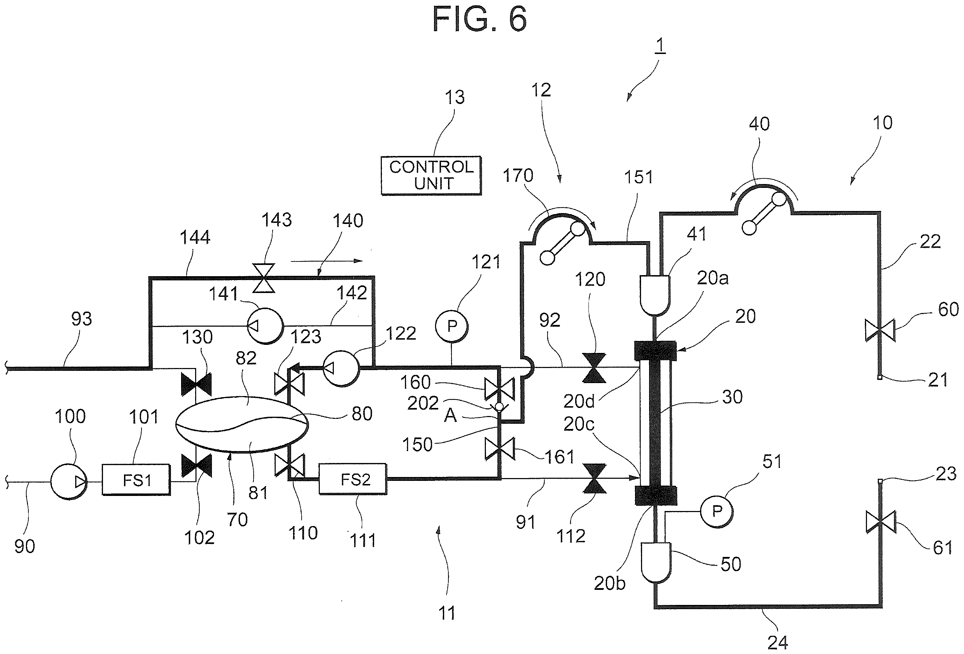

In addition, in a case where a blood pressure of a patient rapidly decreases in a blood purification process, for example, urgent fluid replacement can be performed. In this urgent fluid replacement process, which does not form part of the invention, as illustrated in Fig. 6, for example, the opening/closing valve 160 and the opening/closing valve 161 are opened, and the fluid replacement pump 170 rotates forwardly. In this manner, the dialysate in the second dialysate supply circuit 91 is directly supplied to the blood collection circuit 22 through the bypass circuit 150 and the connection circuit 151 so that the dialysate is urgently supplied to the blood. That is, at this time, the dialysate in the second dialysate supply circuit 91 is distributed to the blood circuit 10 and the first dialysate discharge circuit 92 by using the fluid replacement pump 170.

At this time, the opening/closing valve 143 of the water removing circuit 140 is also opened so that a part of the dialysate that has flowed from the second dialysate supply circuit 91 into the bypass circuit 150 and the dialysate in the second dialysate discharge circuit 93 are supplied to the dialysate discharge chamber 82 of the metering pump 70. The dialysate in the second dialysate discharge circuit 93 flows back in, for example, the second circuit 144 to be supplied to the dialysate discharge chamber 82. In this manner, a supply/discharge balance of the dialysate in the metering pump 70 in urgent fluid replacement (where the amount of dialysate discharged from the dialysate supply chamber 81 needs to be equal to the amount of dialysate flowing into the dialysate discharge chamber 82) can be obtained. At this time, the flow rate of the second dialysate supply circuit 91 is kept at a predetermined value or more so that a detection sensitivity of a flow rate sensor such as the flow rate sensor 111 can be maintained. In addition, the check valve 202 can prevent the dialysate in the first dialysate discharge circuit 92 from reversely flowing in the bypass circuit 150 and being supplied to the blood.

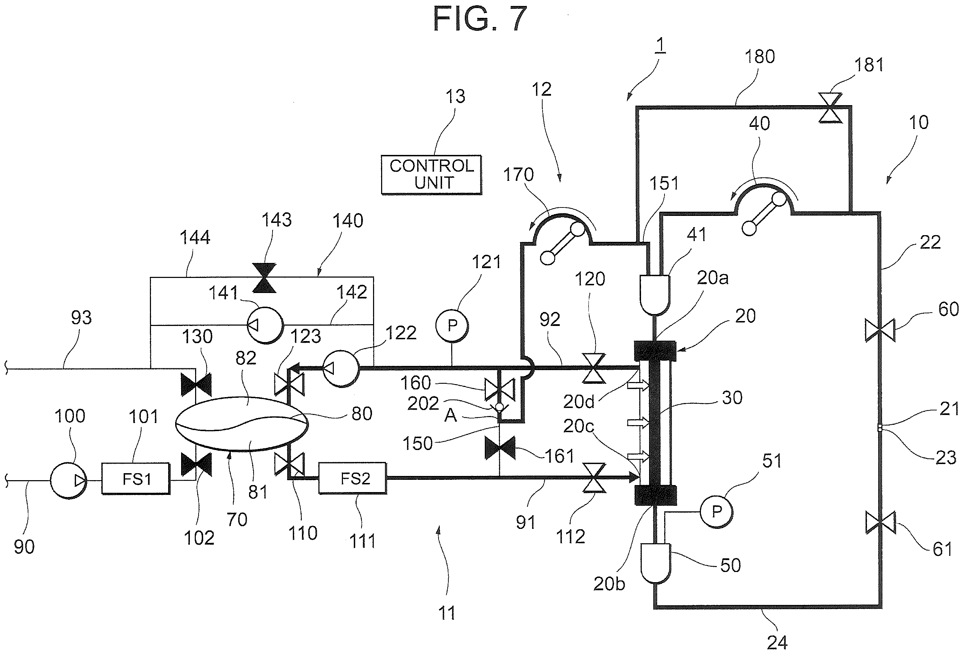

In this embodiment, as illustrated in Fig. 7, the blood purification device 1 may include a blood returning circuit 180. The blood returning circuit 180 connects, for example, a portion of the connection circuit 151 closer to the drip chamber 41 than the fluid replacement pump 170 in the fluid replacement/priming circuit 12 to a portion of the blood collection circuit 22 closer to the blood collection part 21 than the blood pump 40. The blood returning circuit 180 includes an opening/closing valve 181. In performing priming, for example, the clamp valve 60 or 61 is closed, the opening/closing valve 181 is opened, and in a manner similar to that of the embodiment above, the fluid replacement pump 170 is rotated reversely so that dialysate that has flowed out to the primary side of the blood purification membrane 30 is also supplied to the blood returning circuit 180 by reversely rotating the blood pump 40. In this manner, priming can also be performed in the blood returning circuit 180. In returning blood remaining in the blood circuit 10 to the patient after the blood purification process, the opening/closing valve 181 is opened, and at the side of the blood collection part 21, the fluid replacement pump 170 is rotated forwardly and the blood pump 40 is stopped so that the dialysate is supplied through the blood returning circuit 180 to return the blood, whereas at the side of the blood return part 23, the fluid replacement pump 170 and the blood pump 40 are rotated forwardly so that the dialysate is supplied through the blood returning circuit 180 to return the blood to the patient.

### Second Embodiment

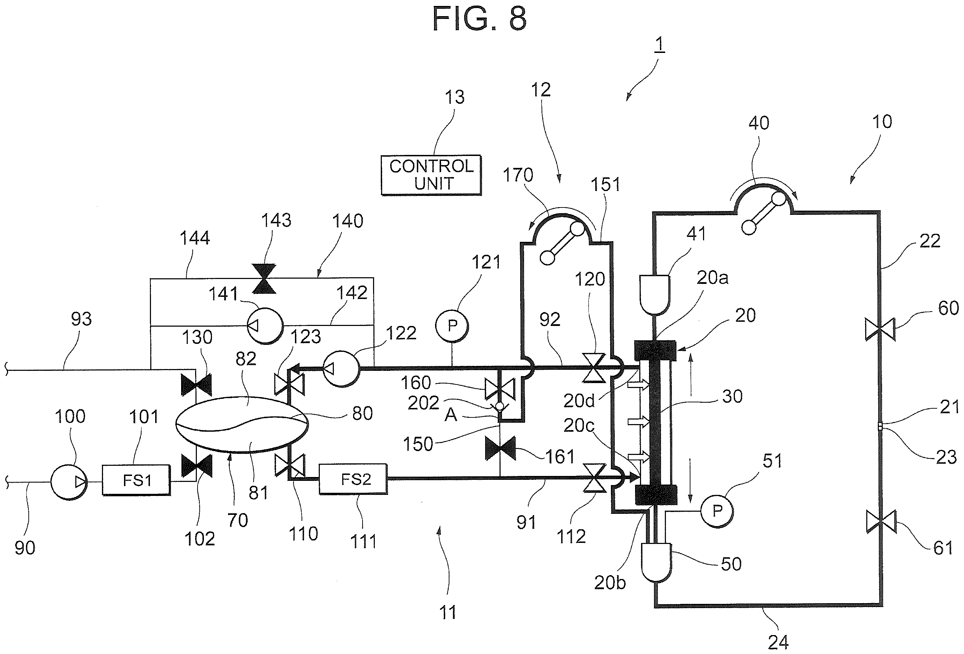

In another embodiment, as illustrated in Fig. 8, the connection circuit 151 of the fluid replacement/priming circuit 12 may be connected to the blood return circuit 24 instead of the blood collection circuit 22. The connection circuit 151 is connected to, for example, the drip chamber 50.

In this case, in the priming first process, while the opening/closing valve 160 is open and the opening/closing valve 161 is closed, the fluid replacement pump 170 is rotated reversely. In the blood circuit 10, while the blood collection part 21 and the blood return part 23 are connected to each other, the blood pump 40 is also rotated reversely. The flow rate of the blood pump 40 is adjusted to be lower than the flow rate of the fluid replacement pump 170. In this manner, dialysate supplied to the secondary side of the blood purification membrane 30 of the blood purification unit 20 flows into the primary side through the blood purification membrane 30, and flows out into the blood collection circuit 22 and the blood return circuit 24 from both the inlet part 20a and the outlet part 20b of the blood purification unit 20. Then, the dialysate is discharged from the drip chamber 50 of the blood return circuit 24 to the first dialysate discharge circuit 92 through the connection circuit 151 and the bypass circuit 150 of the fluid replacement/priming circuit 12.

In the priming first process, the removed bubbles are compressed by the discharge pump 122 through the first dialysate discharge circuit 92 and discharged to the dialysate discharge chamber 82 of the metering pump 70 so that a supply/discharge balance of the dialysate in the metering pump 70 (where the amount of dialysate discharged from the dialysate supply chamber 81 needs to be equal to the amount of dialysate flowing into the dialysate discharge chamber 82) is disturbed and the dialysate circuit 11 comes to have a negative pressure in some cases.

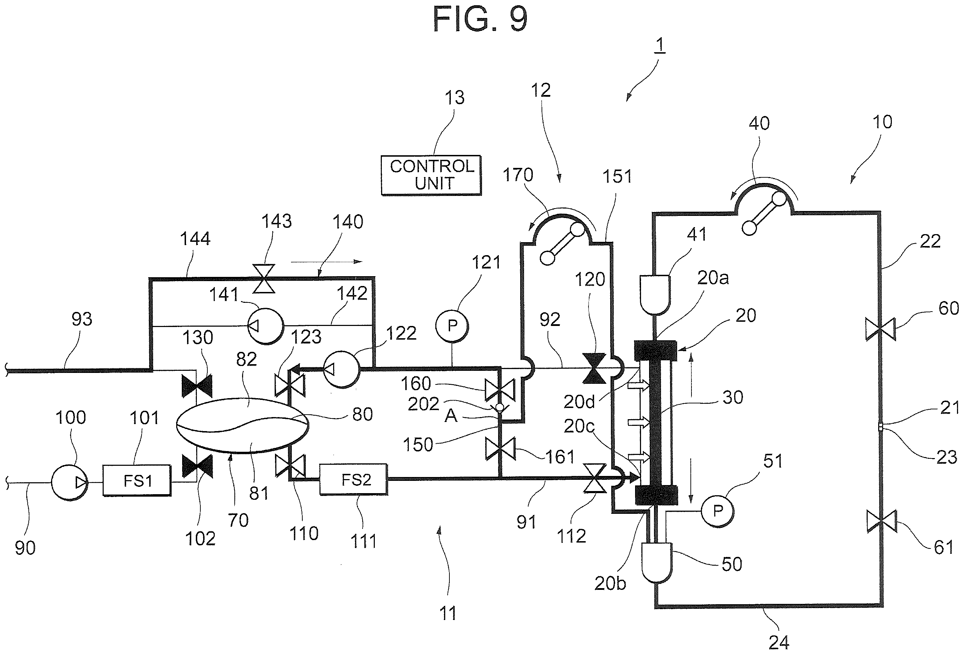

At this time, as illustrated in Fig. 9, for example, while the opening/closing valve 120 is closed and the opening/closing valve 161 is open, the opening/closing valve 143 of the water removing circuit 140 is opened, the dialysate in an amount corresponding to the volume of compressed bubbles reversely flows in the second circuit 144 and is supplied to the dialysate discharge chamber 82. In this manner, a supply/discharge balance of the dialysate in the metering pump 70 in the priming first process (where the amount of dialysate discharged from the dialysate supply chamber 81 needs to be equal to the amount of dialysate flowing into the dialysate discharge chamber 82) can be obtained. At this time, the flow rate of the second dialysate supply circuit 91 is kept at a predetermined value or more so that a detection sensitivity of a flow rate sensor such as the flow rate sensor 111 can be maintained. In addition, the check valve 202 can prevent the dialysate in the first dialysate discharge circuit 92 from reversely flowing in the bypass circuit 150 and being supplied to the secondary side of the blood purification membrane 30 of the blood purification unit 20. Since the opening/closing valve 120 is closed, it is possible to prevent the dialysate in the first dialysate discharge circuit 92 from being supplied to the secondary side of the blood purification membrane 30 of the blood purification unit 20.

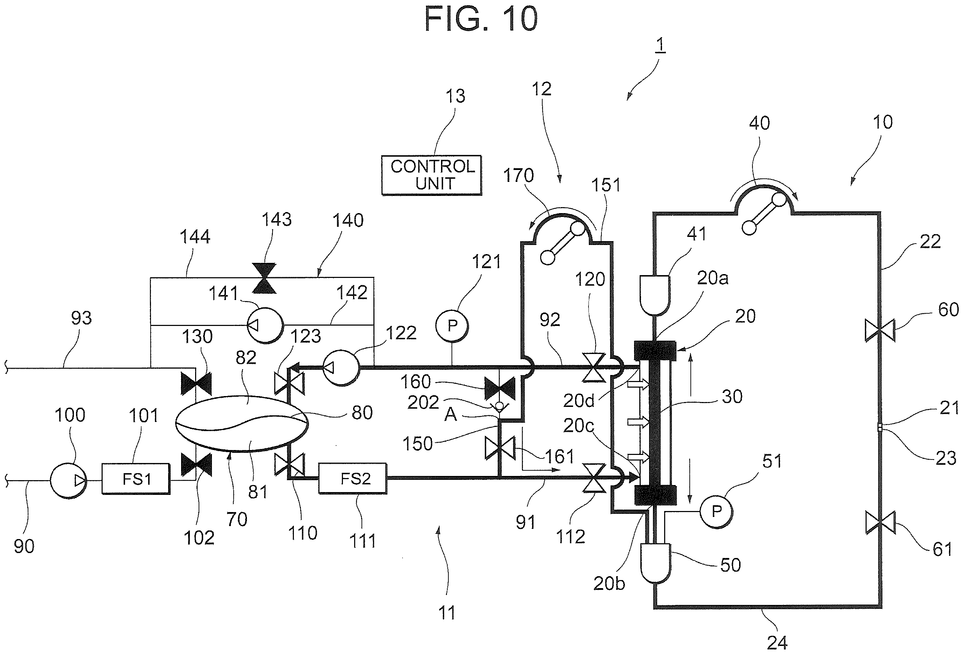

In the priming second process, as illustrated in Fig. 10, the opening/closing valve 160 is closed and the opening/closing valve 161 is opened so that dialysate caused to flow into the fluid replacement/priming circuit 12 by the fluid replacement pump 170 is returned to the second dialysate supply circuit 91 and supplied to the blood purification unit 20 again.

In these priming processes, a flushing operation of opening and closing the clamp valves 60 and 61 may be performed, or the flow rate of the blood pump 40 may be changed to promote removal of bubbles. The flow rate of the blood pump 40 may be higher than the flow rate of the fluid replacement pump 170, or the blood pump 40 may be rotated forwardly.

In the priming processes, based on a result of pressure detection by the pressure sensor 51, the control unit 13, for example, may adjust the flow rate of the fluid replacement pump 170 so that a pressure on the blood purification membrane 30 is within a predetermined range.

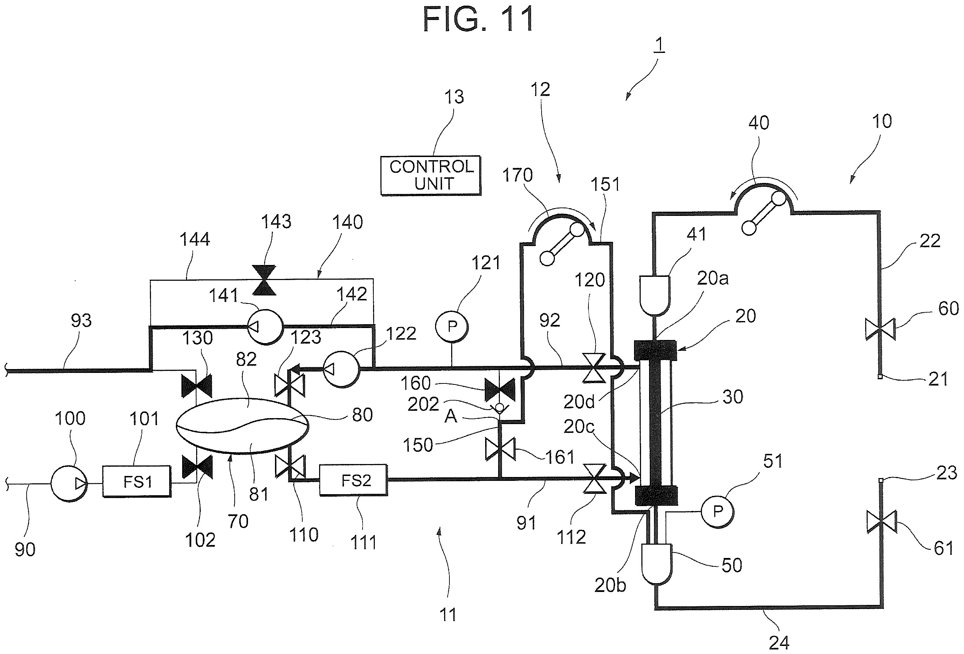

In a blood purification process, which does not form part of the invention, as illustrated in Fig. 11, the blood pump 40 rotates forwardly so that blood circulates in the blood circuit 10. In the dialysate circuit 11, dialysate is supplied from the metering pump 70 to the secondary side of the blood purification membrane 30 of the blood purification unit 20 through the second dialysate supply circuit 91, and the dialysate that has passed through the secondary side of the blood purification membrane 30 is discharged to the metering pump 70 through the first dialysate discharge circuit 92. In the blood purification unit 20, blood flows in the primary side of the blood purification membrane 30 and dialysate flows in the secondary side so that waste products in the blood are discharged to the dialysate through the blood purification membrane 30 and, thereby, the blood is purified.

In the fluid replacement/priming circuit 12, while the opening/closing valve 161 is open and the opening/closing valve 160 is closed, the fluid replacement pump 170 rotates forwardly and the dialysate is suitably supplied to the blood return circuit 24 so that fluid replacement is performed on a patient.

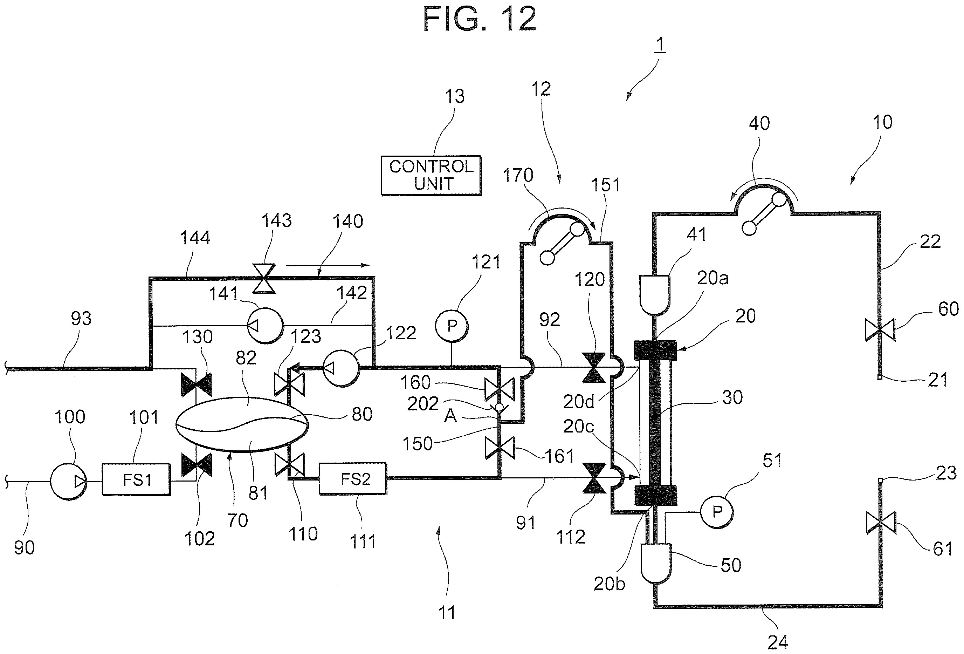

In addition, in a case where a blood pressure of the patient rapidly decreases in a blood purification process, for example, urgent fluid replacement can be performed. In this urgent fluid replacement process, which does not form part of the invention, as illustrated in Fig. 12, for example, the opening/closing valve 160 and the opening/closing valve 161 are opened, and the fluid replacement pump 170 rotates forwardly. In this manner, the dialysate in the second dialysate supply circuit 91 is directly supplied to the blood return circuit 24 through the bypass circuit 150 and the connection circuit 151 so that the dialysate is urgently supplied to the blood. At this time, the opening/closing valve 143 of the water removing circuit 140 is also opened so that a part of the dialysate that has flowed from the second dialysate supply circuit 91 into the bypass circuit 150 and the dialysate in the second dialysate discharge circuit 93 are supplied to the dialysate discharge chamber 82 of the metering pump 70. The dialysate in the second dialysate discharge circuit 93 flows back in, for example, the second circuit 144 to be supplied to the dialysate discharge chamber 82. In this manner, a supply/discharge balance of the dialysate in the metering pump 70 in urgent fluid replacement (where the amount of dialysate discharged from the dialysate supply chamber 81 needs to be equal to the amount of dialysate flowing into the dialysate discharge chamber 82) can be obtained. At this time, the flow rate of the second dialysate supply circuit 91 is kept at a predetermined value or more so that a detection sensitivity of a flow rate sensor such as the flow rate sensor 111 can be maintained. In addition, the check valve 202 can prevent the dialysate in the first dialysate discharge circuit 92 from reversely flowing in the bypass circuit 150 and being supplied to the blood.

### Third Embodiment

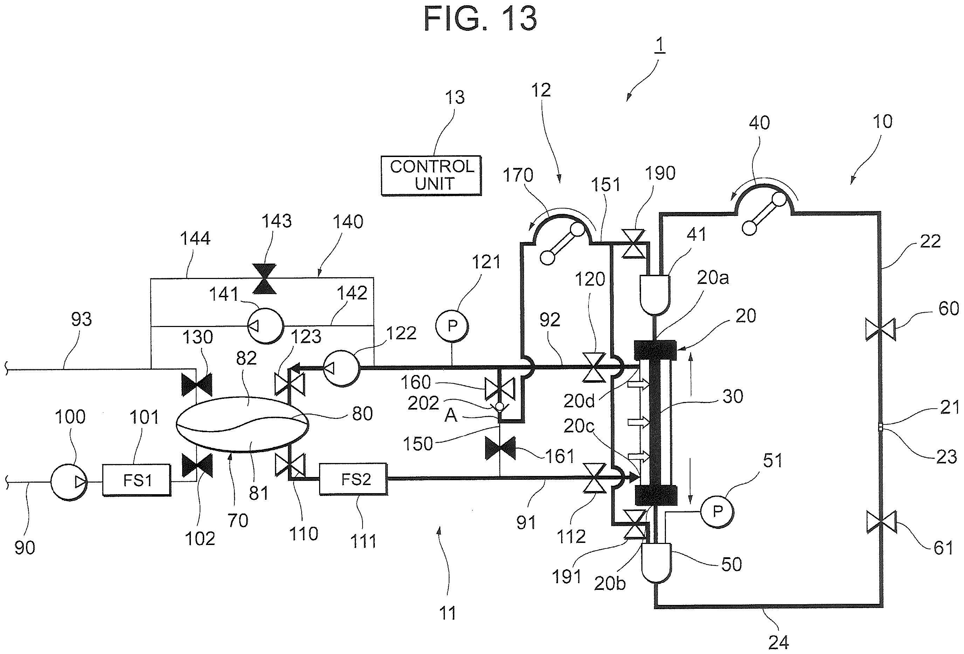

In yet another embodiment, as illustrated in Fig. 13, the connection circuit 151 of the fluid replacement/priming circuit 12 may be connected to both the drip chamber 41 of the blood collection circuit 22 and the drip chamber 50 of the blood return circuit 24. In this case, opening/closing valves 190 and 191 are individually provided at branches of the connection circuit 151.

In such a case, in the priming first process, while the opening/closing valve 160 is open, the opening/closing valve 161 is closed, and both the opening/closing valves 190 and 191 are open, the fluid replacement pump 170 is rotated reversely. The blood pump 40 is rotated forwardly. The flow rate of the blood pump 40 is adjusted to be lower than the flow rate of the fluid replacement pump 170. In this manner, the dialysate supplied to the secondary side of the blood purification membrane 30 of the blood purification unit 20 through the second dialysate supply circuit 91 flows to the primary side through the blood purification membrane 30, and flows out into the blood collection circuit 22 and the blood return circuit 24 from both the inlet part 20a and the outlet part 20b of the blood purification unit 20. Then, the dialysate flows from the drip chamber 41 of the blood collection circuit 22 and the drip chamber 50 of the blood return circuit 24 to the connection circuit 151 of the fluid replacement/priming circuit 12, and is discharged to the first dialysate discharge circuit 92 through the connection circuit 151 and the bypass circuit 150.

In the priming first process, the removed bubbles are compressed by the discharge pump 122 through the first dialysate discharge circuit 92 and discharged to the dialysate discharge chamber 82 of the metering pump 70 so that a supply/discharge balance of the dialysate in the metering pump 70 (where the amount of dialysate discharged from the dialysate supply chamber 81 needs to be equal to the amount of dialysate flowing into the dialysate discharge chamber 82) is disturbed and the dialysate circuit 11 comes to have a negative pressure in some cases.

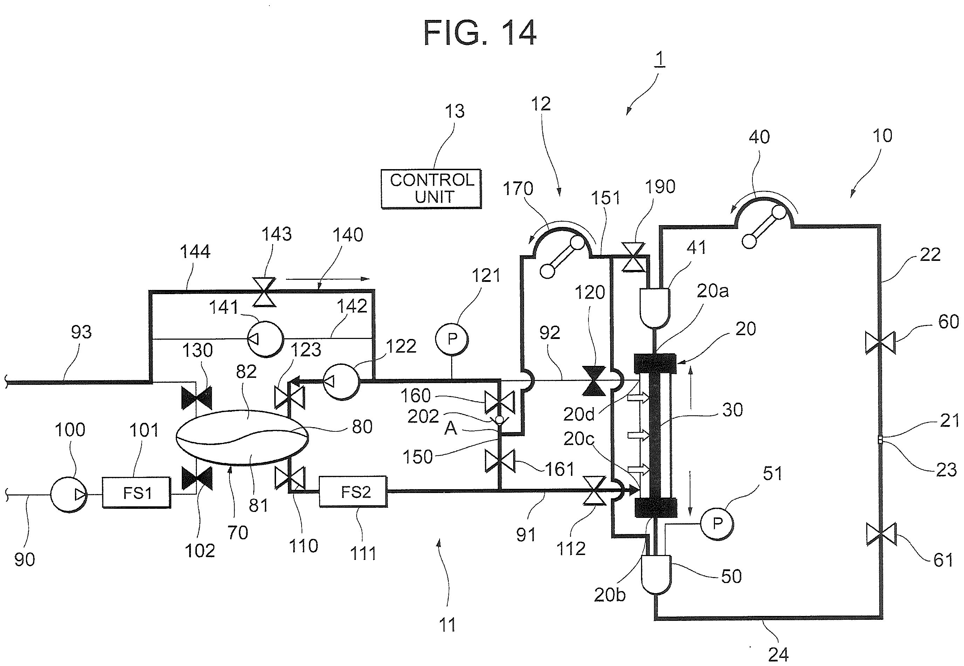

At this time, as illustrated in Fig. 14, for example, while the opening/closing valve 120 is closed and the opening/closing valve 161 is open, the opening/closing valve 143 of the water removing circuit 140 is also opened, the dialysate in an amount corresponding to the volume of the compressed bubbles reversely flows in the second circuit 144 and is supplied to the dialysate discharge chamber 82. In this manner, a supply/discharge balance of the dialysate in the metering pump 70 in the priming first process (where the amount of dialysate discharged from the dialysate supply chamber 81 needs to be equal to the amount of the dialysate flowing into dialysate discharge chamber 82) can be obtained. At this time, the flow rate of the second dialysate supply circuit 91 is kept at a predetermined value or more so that a detection sensitivity of a flow rate sensor such as the flow rate sensor 111 can be maintained. In addition, the check valve 202 can prevent the dialysate in the first dialysate discharge circuit 92 from reversely flowing in the bypass circuit 150 and being supplied to the secondary side of the blood purification membrane 30 of the blood purification unit 20. Since the opening/closing valve 120 is closed, it is possible to prevent the dialysate in the first dialysate discharge circuit 92 from being supplied to the secondary side of the blood purification membrane 30 of the blood purification unit 20.

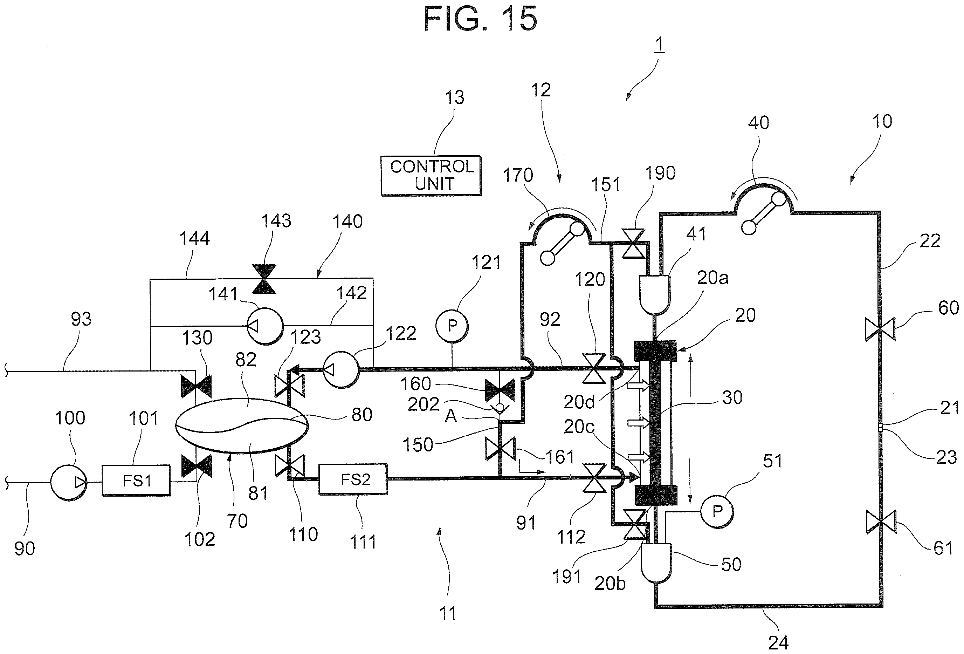

In the priming second process, as illustrated in Fig. 15, the opening/closing valve 161 is opened and the opening/closing valve 160 is closed so that dialysate caused to flow into the fluid replacement/priming circuit 12 is returned to the second dialysate supply circuit 91 and supplied to the blood purification unit 20 again.

In these priming processes, a flushing operation of opening and closing the clamp valves 60 and 61 may be performed, or the flow rate of the blood pump 40 may be changed to promote removal of bubbles. The flow rate of the blood pump 40 may be higher than the flow rate of the fluid replacement pump 170, or the blood pump 40 may be rotated reversely. Only one of the opening/closing valves 190 and 191 may be opened.

In the priming processes, based on a result of pressure detection by the pressure sensor 51, the control unit 13, for example, may adjust the flow rate of the fluid replacement pump 170 so that a pressure on the blood purification membrane 30 is within a predetermined range.

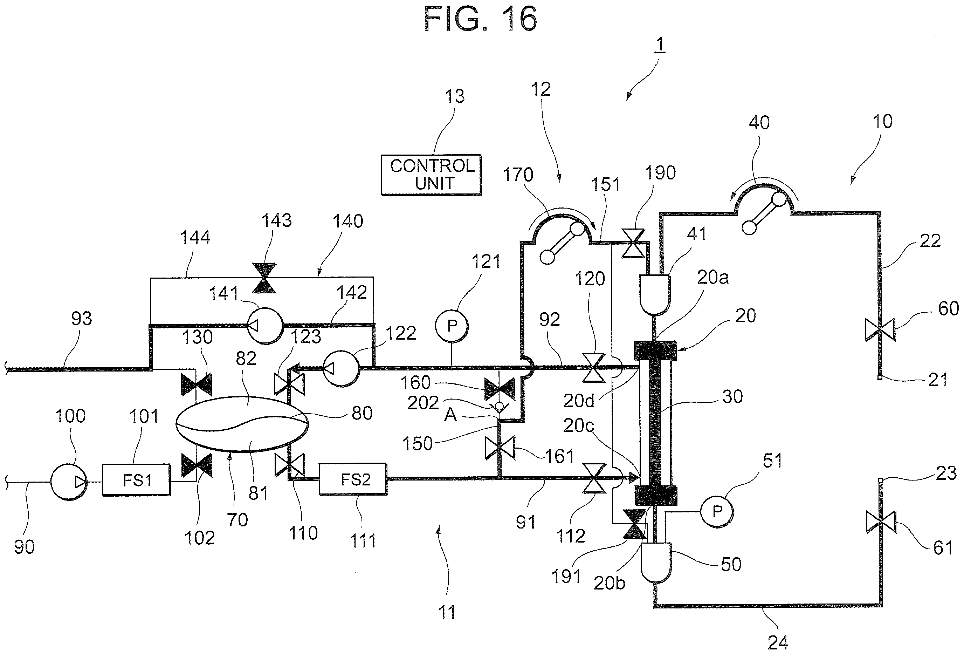

In a blood purification process, which does not form part of the invention, as illustrated in Fig. 16, the blood pump 40 rotates forwardly so that blood circulates in the blood circuit 10. In the dialysate circuit 11, dialysate is supplied from the metering pump 70 to the secondary side of the blood purification membrane 30 of the blood purification unit 20 through the second dialysate supply circuit 91, and the dialysate that has passed through the secondary side of the blood purification membrane 30 is discharged to the metering pump 70 through the first dialysate discharge circuit 92. In the blood purification unit 20, blood flows in the primary side of the blood purification membrane 30 and dialysate flows in the secondary side so that waste products in the blood are discharged to the dialysate through the blood purification membrane 30 and, thereby, the blood is purified.

In the fluid replacement/priming circuit 12, while the opening/closing valve 161 is open, the opening/closing valve 160 is closed, and at least one of the opening/closing valves 190 and 191 is open, the fluid replacement pump 170 rotates forwardly and the dialysate is suitably supplied to the blood collection circuit 22 and the blood return circuit 24 so that fluid replacement is performed on a patient.

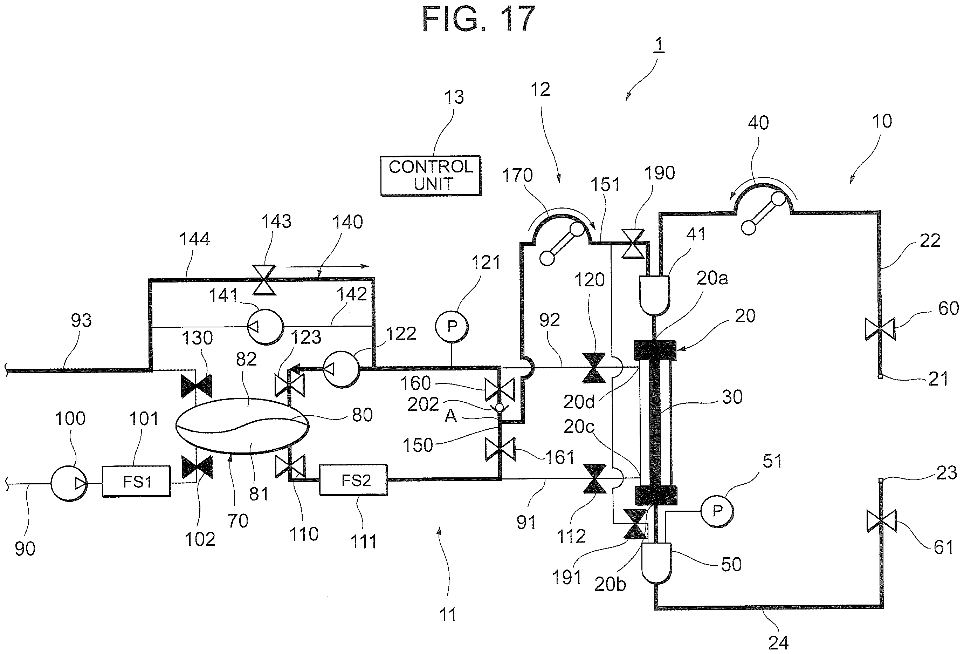

In addition, in a case where a blood pressure of the patient rapidly decreases in a blood purification process, for example, urgent fluid replacement can be performed. In this urgent fluid replacement process, which does not form part of the invention, as illustrated in Fig. 17, for example, the opening/closing valve 160 and the opening/closing valve 161 are opened, and the fluid replacement pump 170 rotates forwardly. In this manner, the dialysate in the second dialysate supply circuit 91 is directly supplied to the blood collection circuit 22 through the bypass circuit 150 and the connection circuit 151 so that the dialysate is urgently supplied to the blood. At this time, the opening/closing valve 143 of the water removing circuit 140 is also opened so that a part of the dialysate that has flowed from the second dialysate supply circuit 91 into the bypass circuit 150 and the dialysate in the second dialysate discharge circuit 93 are supplied to the dialysate discharge chamber 82 of the metering pump 70. The dialysate in the second dialysate discharge circuit 93 flows back in, for example, the second circuit 144 to be supplied to the dialysate discharge chamber 82. In this manner, a supply/discharge balance of the dialysate in the metering pump 70 in urgent fluid replacement (where the amount of dialysate discharged from the dialysate supply chamber 81 needs to be equal to the amount of dialysate flowing into the dialysate discharge chamber 82) can be obtained. At this time, the flow rate of the second dialysate supply circuit 91 is kept at a predetermined value or more so that a detection sensitivity of a flow rate sensor such as the flow rate sensor 111 can be maintained. In addition, the check valve 202 can prevent the dialysate in the first dialysate discharge circuit 92 from reversely flowing in the bypass circuit 150 and being supplied to the blood.

### Fourth Embodiment

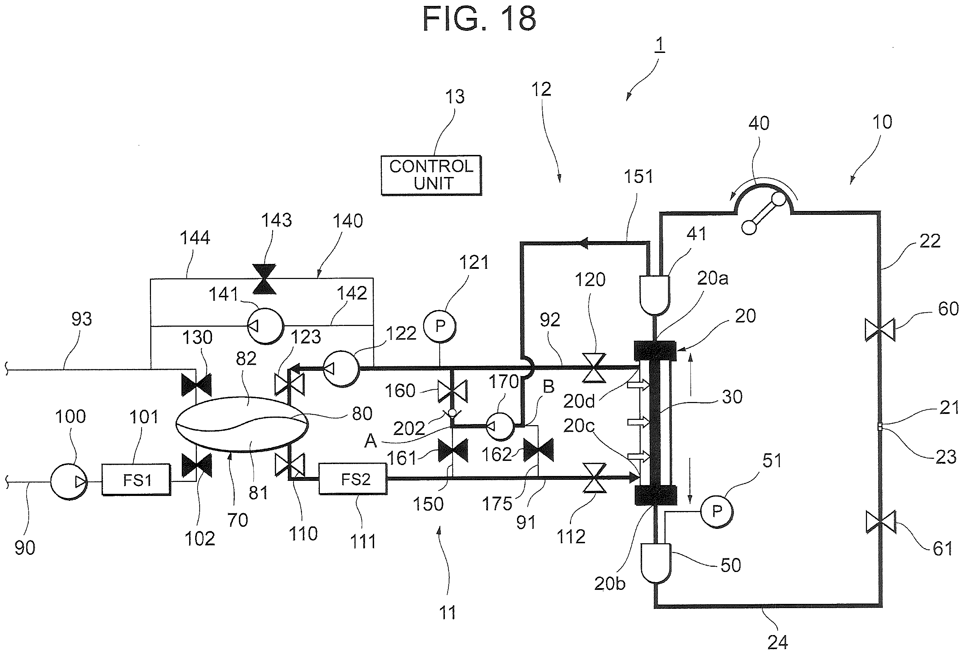

In still another embodiment, as illustrated in Fig. 18, the fluid replacement/priming circuit 12 includes a bypass circuit 150 connecting the second dialysate supply circuit 91 to the first dialysate discharge circuit 92, a sub-bypass circuit 175 connecting an intermediate portion of the bypass circuit 150 to the second dialysate supply circuit 91, and a connection circuit 151 connecting the blood circuit 10 to an intermediate portion of the sub-bypass circuit 175. The bypass circuit 150 includes an opening/closing valve 160 (first flow rate adjusting unit) disposed between a connection point A to the sub-bypass circuit 175 and a connection point to the first dialysate discharge circuit 92, and an opening/closing valve 161 (second flow rate adjusting unit) disposed between a connection point A to the sub-bypass circuit 175 and a connection point to the second dialysate supply circuit 91. The bypass circuit 150 includes a check valve 202. The fluid replacement pump 170 is disposed between a connection point A between the sub-bypass circuit 175 and the bypass circuit 150 and a connection point B between the sub-bypass circuit 175 and the connection circuit 151. The sub-bypass circuit 175 includes an opening/closing valve 162 (third flow rate adjusting unit) between the connection point B to the connection circuit 151 and a connection point to the second dialysate supply circuit 91.

In this case, in the priming first process, while the opening/closing valve 160 is open and the opening/closing valves 161 and 162 are closed, the fluid replacement pump 170 is rotated reversely. In the blood circuit 10, while the blood collection part 21 and the blood return part 23 are connected to each other, the blood pump 40 is rotated forwardly. The flow rate of the blood pump 40 is adjusted to be lower than the flow rate of the fluid replacement pump 170. In this manner, the dialysate supplied to the secondary side of the blood purification membrane 30 of the blood purification unit 20 flows into the primary side through the blood purification membrane 30, and flows out into the blood collection circuit 22 and the blood return circuit 24 from both the inlet part 20a and the outlet part 20b of the blood purification unit 20. Then, the dialysate is discharged from the drip chamber 41 of the blood collection circuit 22 to the first dialysate discharge circuit 92 through the connection circuit 151, the sub-bypass circuit 175, and the bypass circuit 150 of the fluid replacement/priming circuit 12.

In the priming first process, the removed bubbles are compressed by the discharge pump 122 through the first dialysate discharge circuit 92 and discharged to the dialysate discharge chamber 82 of the metering pump 70 so that a supply/discharge balance of the dialysate in the metering pump 70 (where the amount of dialysate discharged from the dialysate supply chamber 81 needs to be equal to the amount of dialysate flowing into the dialysate discharge chamber 82) is disturbed and the dialysate circuit 11 comes to have a negative pressure in some cases.

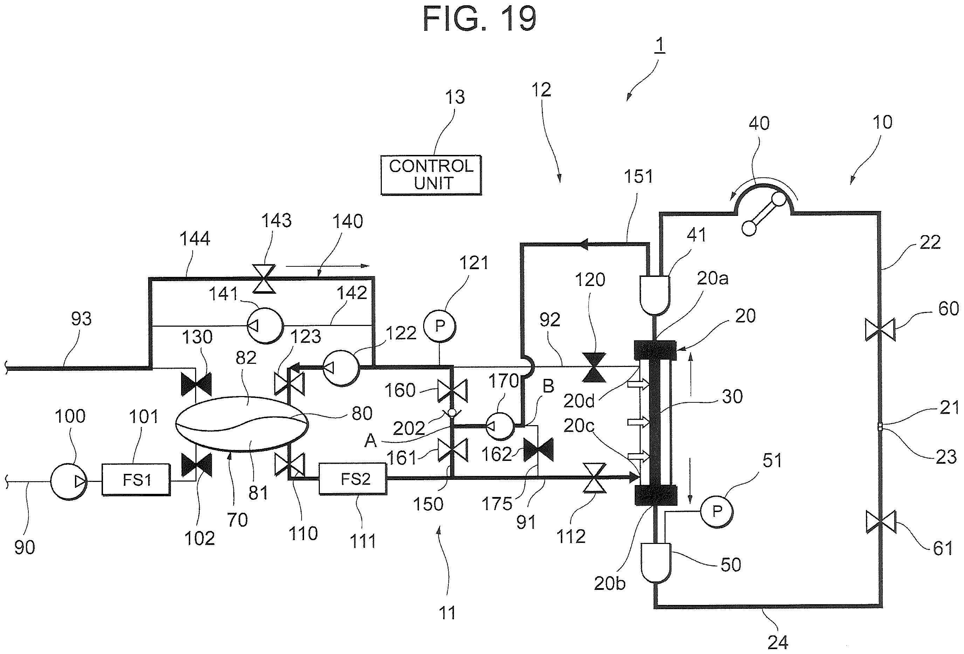

At this time, as illustrated in Fig. 19, for example, while the opening/closing valve 120 is closed and the opening/closing valve 161 is open, the opening/closing valve 143 of the water removing circuit 140 is opened, the dialysate in an amount corresponding to the volume of the compressed bubbles reversely flows in, for example, the second circuit 144 and is supplied to the dialysate discharge chamber 82. In this manner, a supply/discharge balance of the dialysate in the metering pump 70 in the priming first process (where the amount of dialysate discharged from the dialysate supply chamber 81 needs to be equal to the amount of dialysate flowing into the dialysate discharge chamber 82) can be obtained. At this time, the flow rate of the second dialysate supply circuit 91 is kept at a predetermined value or more so that a detection sensitivity of a flow rate sensor such as the flow rate sensor 111 can be maintained. In addition, the check valve 202 can prevent the dialysate in the first dialysate discharge circuit 92 from reversely flowing in the bypass circuit 150 and being supplied to the secondary side of the blood purification membrane 30 of the blood purification unit 20. Since the opening/closing valve 120 is closed, it is possible to prevent the dialysate in the first dialysate discharge circuit 92 from being supplied to the secondary side of the blood purification membrane 30 of the blood purification unit 20.

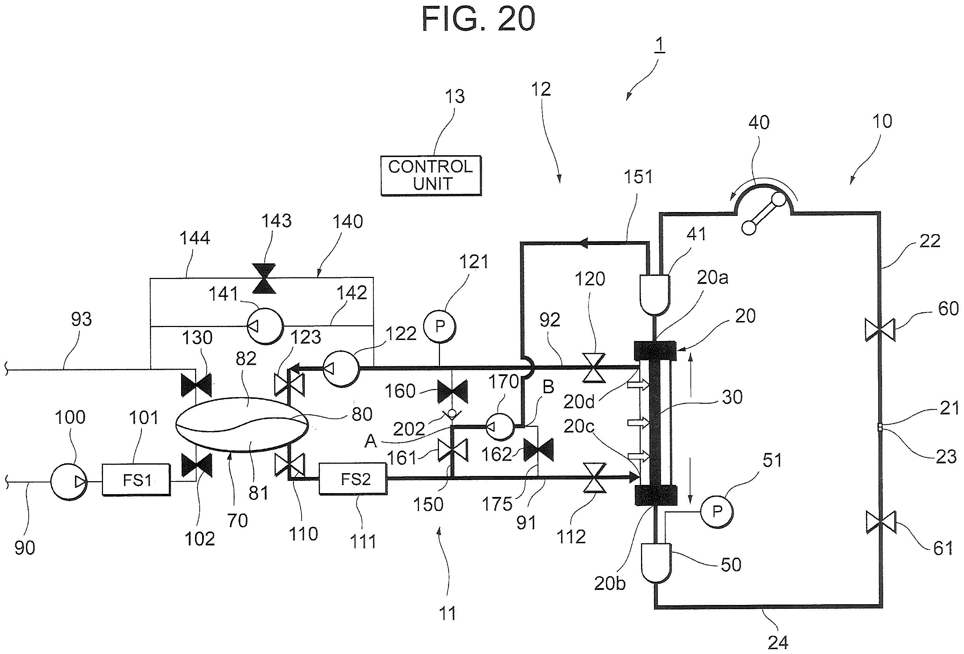

In the priming second process, as illustrated in Fig. 20, the opening/closing valve 160 is closed and the opening/closing valve 161 is opened so that dialysate caused to flow into the fluid replacement/priming circuit 12 by the fluid replacement pump 170 is returned to the second dialysate supply circuit 91 and supplied to the blood purification unit 20 again.

In these priming processes, a flushing operation of opening and closing the clamp valves 60 and 61 may be performed, or the flow rate of the blood pump 40 may be changed to promote removal of bubbles. The flow rate of the blood pump 40 may be higher than the flow rate of the fluid replacement pump 170, or the blood pump 40 may be rotated reversely.

In the priming processes, based on a result of pressure detection by the pressure sensor 51, the control unit 13, for example, may adjust the flow rate of the fluid replacement pump 170 so that a pressure on the blood purification membrane 30 is within a predetermined range.

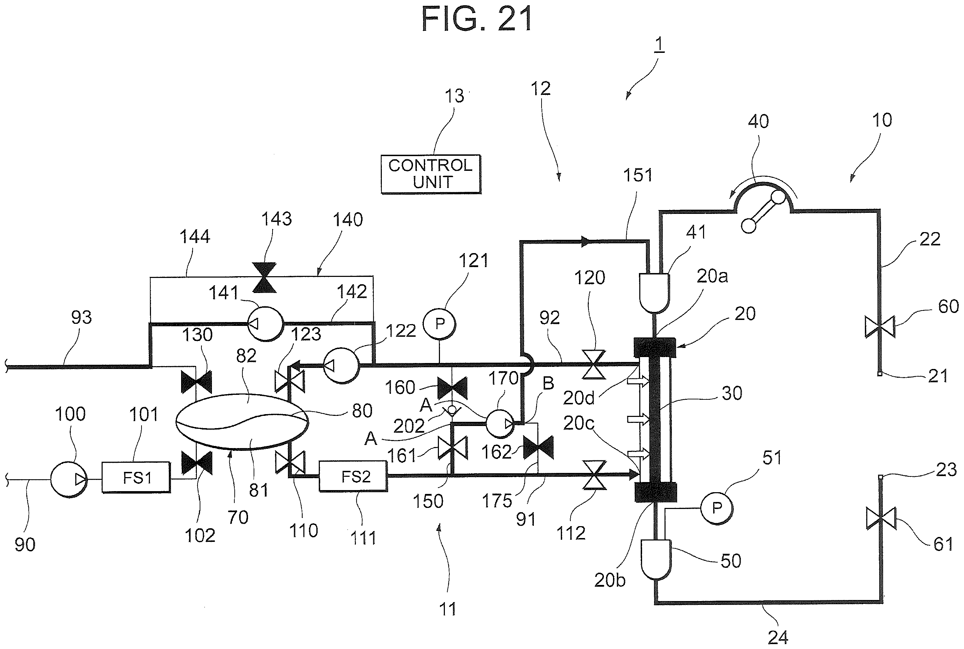

In a blood purification process, which does not form part of the invention, as illustrated in Fig. 21, the blood pump 40 rotates forwardly so that blood circulates in the blood circuit 10. In the dialysate circuit 11, dialysate is supplied from the metering pump 70 to the secondary side of the blood purification membrane 30 of the blood purification unit 20 through the second dialysate supply circuit 91, and the dialysate that has passed through the secondary side of the blood purification membrane 30 is discharged to the metering pump 70 through the first dialysate discharge circuit 92. In the blood purification unit 20, blood flows in the primary side of the blood purification membrane 30 and dialysate flows in the secondary side so that waste products in the blood are discharged to the dialysate through the blood purification membrane 30 and, thereby, the blood is purified.

In the fluid replacement/priming circuit 12, while the opening/closing valve 161 is open and the opening/closing valves 160 and 162 are closed, the fluid replacement pump 170 rotates forwardly so that the dialysate is suitably supplied to the blood collection circuit 22, thereby performing fluid replacement on the patient.

In a case where the connection circuit 151 is disconnected from the sub-bypass circuit 175, the opening/closing valve 160 is closed, the opening/closing valve 161 and the opening/closing valve 162 are opened, and the fluid replacement pump 170 is rotated forwardly, for example, the dialysate in the second dialysate supply circuit 91 flows into the first dialysate discharge circuit 92 through the bypass circuit 150 and the sub-bypass circuit 175. Thus, in this embodiment, it is possible to clean a channel in the fluid replacement pump 170 by using the dialysate. As a result, the channel in the fluid replacement pump 170 does not need to be discarded after each blood purification process, and can be incorporated in the body of the blood purification device 1.

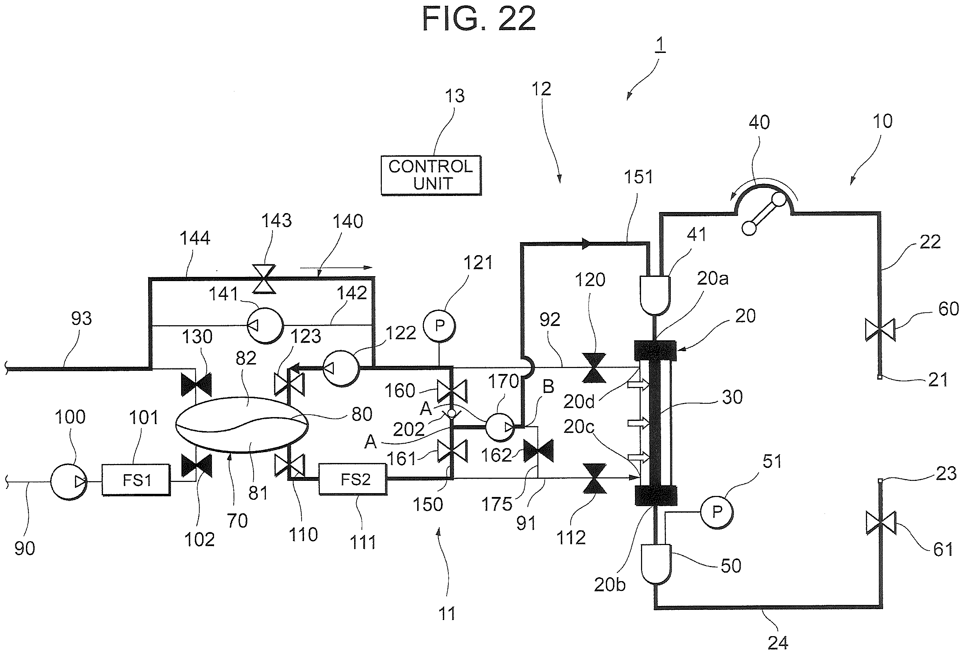

In addition, in a case where a blood pressure of the patient rapidly decreases in a blood purification process, for example, urgent fluid replacement can be performed. In this urgent fluid replacement process, which does not form part of the invention, as illustrated in Fig. 22, for example, while the opening/closing valve 160 and the opening/closing valve 161 are opened and the opening/closing valve 162 is closed, the fluid replacement pump 170 rotates forwardly. In this manner, the dialysate in the second dialysate supply circuit 91 is directly supplied to the blood collection circuit 22 through the bypass circuit 150, the sub-bypass circuit 175, and the connection circuit 151 so that the dialysate is urgently supplied to the blood. At this time, the opening/closing valve 143 of the water removing circuit 140 is also opened so that a part of the dialysate that has flowed from the second dialysate supply circuit 91 into the bypass circuit 150 and the dialysate in the second dialysate discharge circuit 93 are supplied to the dialysate discharge chamber 82 of the metering pump 70. The dialysate in the second dialysate discharge circuit 93 flows back in, for example, the second circuit 144 to be supplied to the dialysate discharge chamber 82. In this manner, a supply/discharge balance of the dialysate in the metering pump 70 in urgent fluid replacement (where the amount of dialysate discharged from the dialysate supply chamber 81 needs to be equal to the amount of dialysate flowing into the dialysate discharge chamber 82) can be obtained. At this time, the flow rate of the second dialysate supply circuit 91 is kept at a predetermined value or more so that a detection sensitivity of a flow rate sensor such as the flow rate sensor 111 can be maintained. In addition, the check valve 202 can prevent the dialysate in the first dialysate discharge circuit 92 from reversely flowing in the bypass circuit 150 and being supplied to the blood.

### Fifth Embodiment

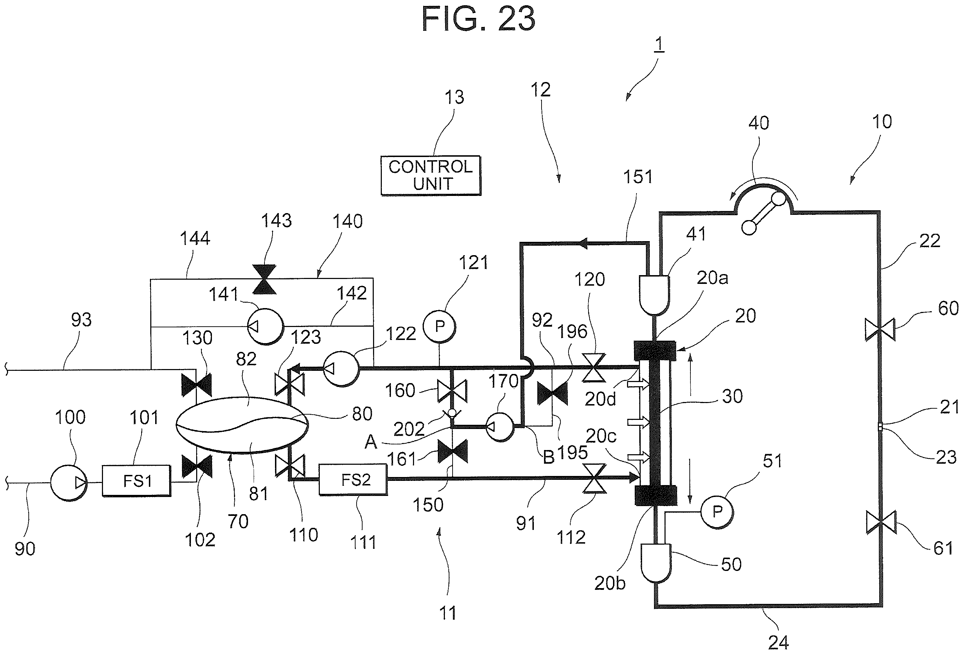

In yet another embodiment, as illustrated in Fig. 23, the fluid replacement/priming circuit 12 includes a bypass circuit 150 connecting the second dialysate supply circuit 91 to the first dialysate discharge circuit 92, a sub-bypass circuit 195 connecting an intermediate portion of the bypass circuit 150 to the first dialysate discharge circuit 92, and a connection circuit 151 connecting the blood circuit 10 to an intermediate portion of the sub-bypass circuit 195. The bypass circuit 150 includes an opening/closing valve 160 (first flow rate adjusting unit) disposed between a connection point A to the sub-bypass circuit 195 and a connection point to the first dialysate discharge circuit 92, and an opening/closing valve 161 (second flow rate adjusting unit) disposed between the connection point A to the sub-bypass circuit 195 and a connection point to the second dialysate supply circuit 91. The bypass circuit 150 includes a check valve 202. The fluid replacement pump 170 is disposed between the connection point A between the sub-bypass circuit 195 and the bypass circuit 150 and a connection point B between the sub-bypass circuit 195 and the connection circuit 151. The sub-bypass circuit 195 includes an opening/closing valve 196 (third flow rate adjusting unit) between the connection point B to the connection circuit 151 and the connection point to the first dialysate discharge circuit 92.

In such a case, in the priming first process, while both the opening/closing valve 161 and the opening/closing valve 196 are closed and the opening/closing valve 160 is open, the fluid replacement pump 170 is rotated reversely. The blood pump 40 is rotated forwardly. The flow rate of the blood pump 40 is adjusted to be lower than the flow rate of the fluid replacement pump 170. In this manner, the dialysate supplied to the secondary side of the blood purification membrane 30 of the blood purification unit 20 through the second dialysate supply circuit 91 flows into the primary side through the blood purification membrane 30, and flows out to the blood collection circuit 22 and the blood return circuit 24 from both the inlet part 20a and the outlet part 20b of the blood purification unit 20. Then, the dialysate flows from the drip chamber 41 of the blood collection circuit 22 into the connection circuit 151 of the fluid replacement/priming circuit 12, and is discharged to the first dialysate discharge circuit 92 through the sub-bypass circuit 195 and the bypass circuit 150.

In the priming first process, removed bubbles are compressed by the discharge pump 122 through the first dialysate discharge circuit 92 and discharged to the dialysate discharge chamber 82 of the metering pump 70 so that a supply/discharge balance of the dialysate in the metering pump 70 (where the amount of dialysate discharged from the dialysate supply chamber 81 needs to be equal to the amount of dialysate flowing into the dialysate discharge chamber 82) is disturbed and the dialysate circuit 11 comes to have a negative pressure in some cases.

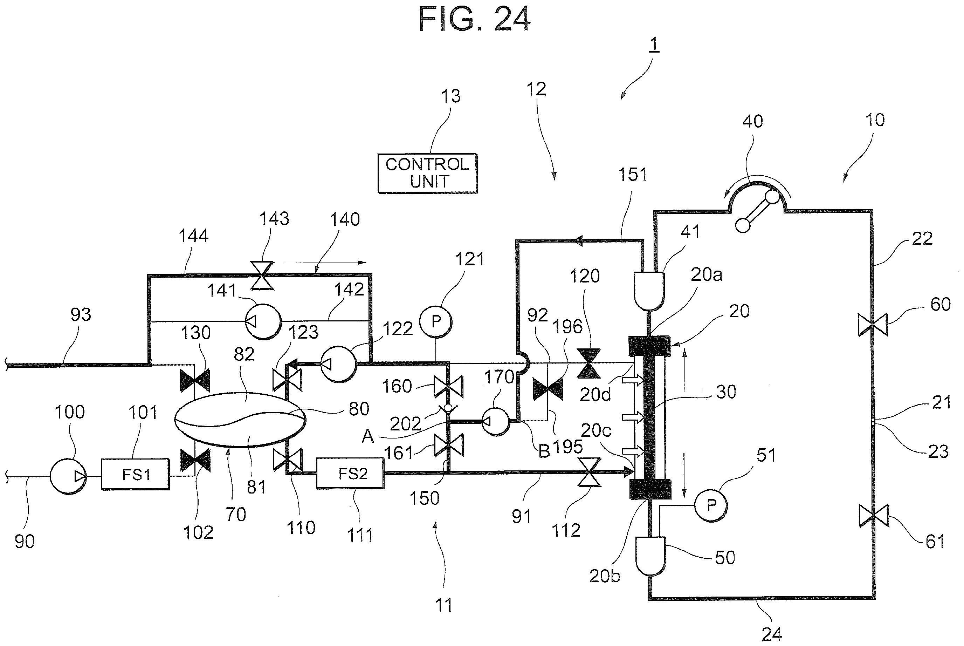

At this time, as illustrated in Fig. 24, for example, while the opening/closing valve 120 is closed and the opening/closing valve 161 is open, the opening/closing valve 143 of the water removing circuit 140 is also opened, the dialysate in an amount corresponding to the volume of the compressed bubbles reversely flows in, for example, the second circuit 144 and is supplied to the dialysate discharge chamber 82. In this manner, a supply/discharge balance of the dialysate in the metering pump 70 in the priming first process (where the amount of dialysate discharged from the dialysate supply chamber 81 needs to be equal to the amount of dialysate flowing into the dialysate discharge chamber 82) can be obtained. At this time, the flow rate of the second dialysate supply circuit 91 is kept at a predetermined value or more so that a detection sensitivity of a flow rate sensor such as the flow rate sensor 111 can be maintained. In addition, the check valve 202 can prevent the dialysate in the first dialysate discharge circuit 92 from reversely flowing in the bypass circuit 150 and being supplied to the secondary side of the blood purification membrane 30 of the blood purification unit 20. Since the opening/closing valve 120 is closed, it is possible to prevent the dialysate in the first dialysate discharge circuit 92 from being supplied to the secondary side of the blood purification membrane 30 of the blood purification unit 20.

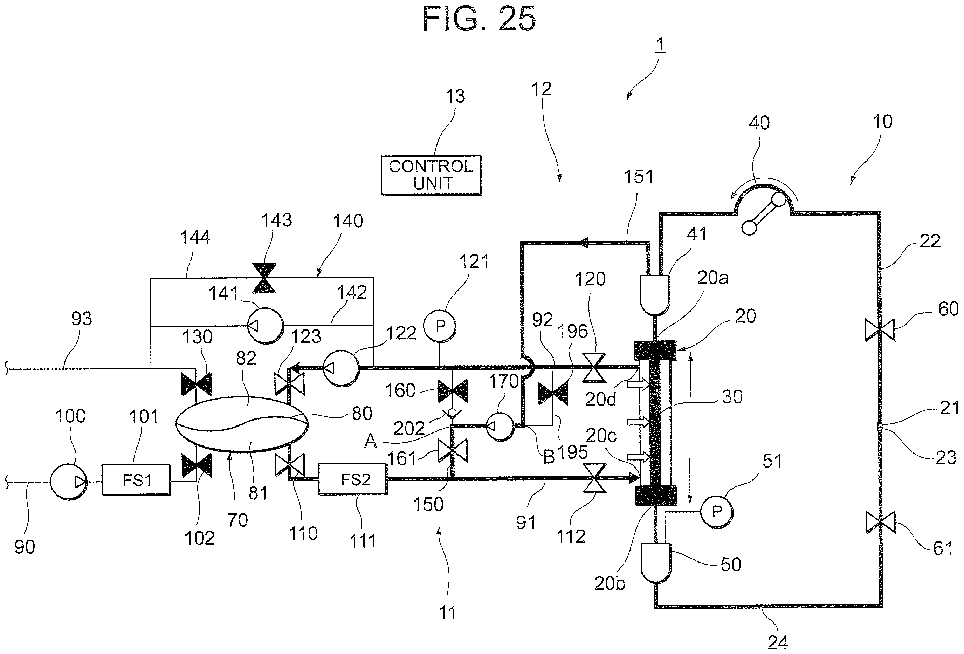

In the priming second process, as illustrated in Fig. 25, the opening/closing valve 160 and the opening/closing valve 196 are closed, the opening/closing valve 161 is opened, and the fluid replacement pump 170 is rotated reversely so that dialysate caused to flow into the fluid replacement/priming circuit 12 is returned to the second dialysate supply circuit 91 and supplied to the blood purification unit 20 again.

In these priming processes, a flushing operation of opening and closing the clamp valves 60 and 61 may be performed, or the flow rate of the blood pump 40 may be changed to promote removal of bubbles. The flow rate of the blood pump 40 may be higher than the flow rate of the fluid replacement pump 170, or the blood pump 40 may be rotated forwardly.

In the priming processes, based on a result of pressure detection by the pressure sensor 51, the control unit 13, for example, may adjust the flow rate of the fluid replacement pump 170 so that a pressure on the blood purification membrane 30 is within a predetermined value.

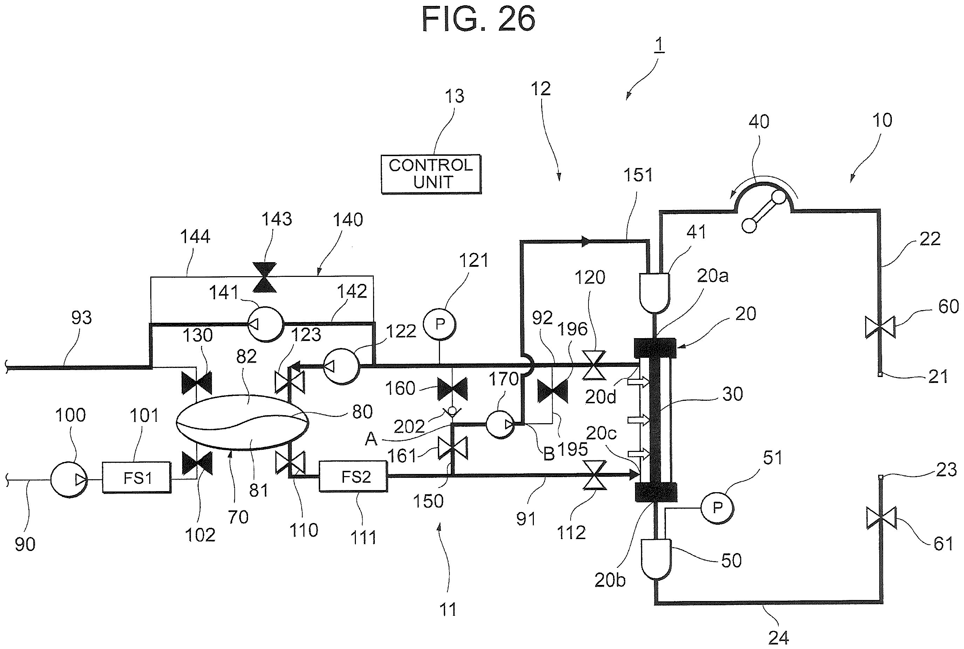

In a blood purification process, which does not form part of the invention, as illustrated in Fig. 26, the blood pump 40 rotates forwardly so that blood circulates in the blood circuit 10. In the dialysate circuit 11, dialysate is supplied from the metering pump 70 to the secondary side of the blood purification membrane 30 of the blood purification unit 20 through the second dialysate supply circuit 91, and the dialysate that has passed through the secondary side of the blood purification membrane 30 is discharged to the metering pump 70 through the first dialysate discharge circuit 92. In the blood purification unit 20, blood flows in the primary side of the blood purification membrane 30 and dialysate flows in the secondary side so that waste products in the blood are discharged to the dialysate through the blood purification membrane 30 and, thereby, the blood is purified.

In the fluid replacement/priming circuit 12, while the opening/closing valve 161 is open, the opening/closing valves 160 and 196 are closed, the fluid replacement pump 170 rotates forwardly so that the dialysate can be suitably supplied to the blood collection circuit 22 through the bypass circuit 150, the sub-bypass circuit 195, and the connection circuit 151, thereby performing fluid replacement on a patient.

In a case where the connection circuit 151 is disconnected from the circuit, the opening/closing valve 160 is closed, the opening/closing valve 161 and the opening/closing valve 196 are opened, and the fluid replacement pump 170 is rotated forwardly, for example, the dialysate in the second dialysate supply circuit 91 flows into the first dialysate discharge circuit 92 through the fluid replacement pump 170 of the bypass circuit 150. Thus, in this embodiment, it is possible to clean a channel in the fluid replacement pump 170 by using the dialysate. As a result, the channel in the fluid replacement pump 170 does not need to be discarded after each blood purification process, and can be incorporated in the body of the blood purification device 1.

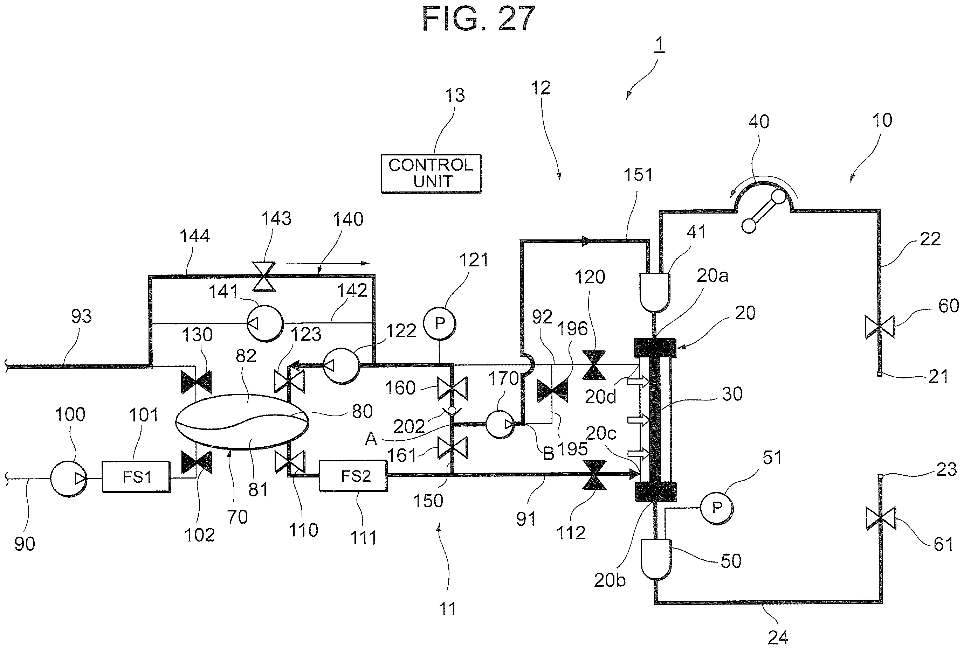

In addition, in a case where a blood pressure of the patient rapidly decreases in a blood purification process, for example, urgent fluid replacement can be performed. In this urgent fluid replacement process, which does not form part of the invention, as illustrated in Fig. 27, for example, the opening/closing valves 161 and 160 are opened, the opening/closing valve 196, the opening/closing valve 112, and the opening/closing valve 120 are closed, and the fluid replacement pump 170 rotates forwardly. In this manner, the dialysate in the second dialysate supply circuit 91 is directly supplied to the blood collection circuit 22 through the bypass circuit 150, the sub-bypass circuit 195, and the connection circuit 151 so that the dialysate is urgently supplied to the blood.

At this time, the opening/closing valve 143 of the water removing circuit 140 is also opened so that a part of the dialysate that has flowed from the second dialysate supply circuit 91 into the bypass circuit 150 and the dialysate in the second dialysate discharge circuit 93 are supplied to the dialysate discharge chamber 82 of the metering pump 70. The dialysate in the second dialysate discharge circuit 93 flows back in, for example, the second circuit 144 to be supplied to the dialysate discharge chamber 82. In this manner, a supply/discharge balance of the dialysate in the metering pump 70 in urgent fluid replacement (where the amount of dialysate discharged from the dialysate supply chamber 81 needs to be equal to the amount of dialysate flowing into the dialysate discharge chamber 82) can be obtained. At this time, the flow rate of the second dialysate supply circuit 91 is kept at a predetermined value or more so that a detection sensitivity of a flow rate sensor such as the flow rate sensor 111 can be maintained. In addition, the check valve 202 can prevent the dialysate in the first dialysate discharge circuit 92 from reversely flowing in the bypass circuit 150 and being supplied to the blood.

### Sixth Embodiment

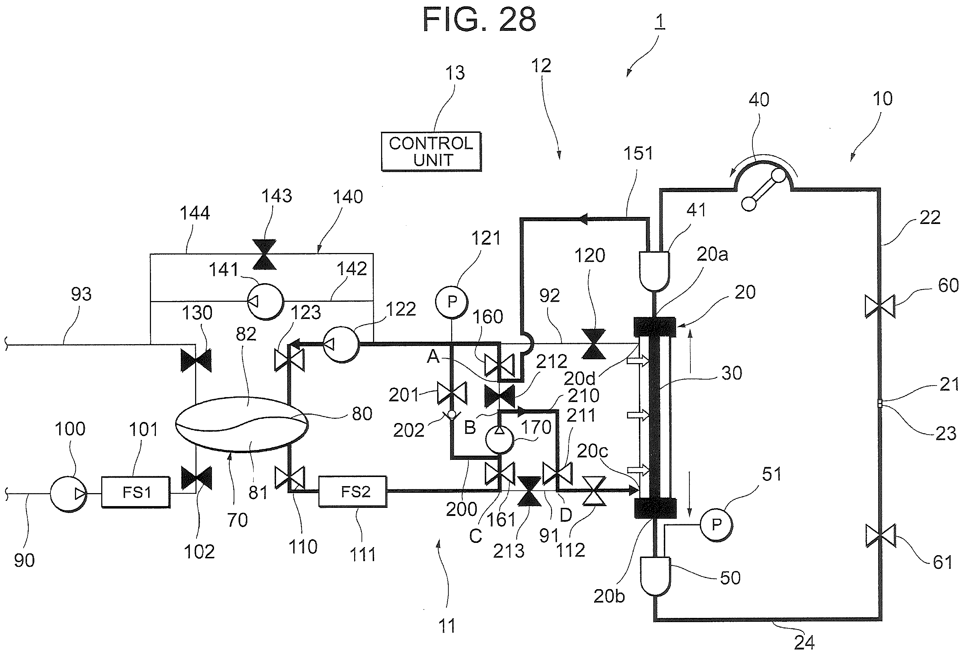

In still another embodiment, as illustrated in Fig. 28, the fluid replacement/priming circuit 12 includes a bypass circuit 150 connecting the second dialysate supply circuit 91 to the first dialysate discharge circuit 92, and a connection circuit 151 connecting the blood circuit 10 to an intermediate portion of the bypass circuit 150. The bypass circuit 150 includes an opening/closing valve 160 (first flow rate adjusting unit) disposed between a connection point A to the connection circuit 151 and a connection point to the first dialysate discharge circuit 92, and an opening/closing valve 161 (third flow rate adjusting unit) and an opening/closing valve 212 (second flow rate adjusting unit) disposed between the connection point A to the connection circuit 151 and a connection point to the second dialysate supply circuit 91. The opening/closing valve 212 is disposed closer to the opening/closing valve 160 than the opening/closing valve 161. The fluid replacement pump 170 is disposed, for example, between the opening/closing valve 212 and the opening/closing valve 161. A sub-bypass circuit 200 connecting a portion of the bypass circuit 150 between the opening/closing valve 212 and the fluid replacement pump 170 to the first dialysate discharge circuit 92 is provided. The sub-bypass circuit 200 includes, for example, an opening/closing valve 201 (sixth flow rate adjusting unit) and a check valve 202. A sub-circuit 210 connecting a connection point B on the bypass circuit 150 between the opening/closing valve 160 and the fluid replacement pump 170 to the second dialysate supply circuit 91 is provided. The sub-circuit 210 includes an opening/closing valve 211 (fourth flow rate adjusting unit), and the bypass circuit 150 includes an opening/closing valve 212 (second flow rate adjusting unit) disposed between the contact point A and the connection point B. The second dialysate supply circuit 91 includes an opening/closing valve 213 (fifth flow rate adjusting unit) disposed between a branch point C to the bypass circuit 150 and a branch point D to the sub-circuit 210.

In this case, in the priming first process, while the opening/closing valve 160 is open, the opening/closing valve 161 (third flow rate adjusting unit), the opening/closing valve 201 (sixth flow rate adjusting unit), and the opening/closing valve 211 are open, and the opening/closing valve 212 and the opening/closing valve 213 are closed, the fluid replacement pump 170 is rotated forwardly. The blood pump 40 is rotated forwardly. The flow rate of the blood pump 40 is adjusted to be lower than the flow rate of the fluid replacement pump 170. In this manner, dialysate flowing from the metering pump 70 through the second dialysate supply circuit 91 flows into the bypass circuit 150, flows into the second dialysate supply circuit 91 through the sub-circuit 210, and is supplied to the secondary side of the blood purification membrane 30 of the blood purification unit 20. Then, the dialysate flows into the primary side through the blood purification membrane 30, and flows out to the blood collection circuit 22 and the blood return circuit 24 from both the inlet part 20a and the outlet part 20b of the blood purification unit 20. Thereafter, the dialysate flows from the drip chamber 41 of the blood collection circuit 22 into the connection circuit 151 of the fluid replacement/priming circuit 12, and is discharged to the first dialysate discharge circuit 92 through a part of the bypass circuit 150.

A part of the dialysate that has flowed from the second dialysate supply circuit 91 into the bypass circuit 150 is discharged to the first dialysate discharge circuit 92 through the sub-bypass circuit 200. In this manner, the dialysate in an amount corresponding to a difference between the flow rate of the dialysate flowing from the metering pump 70 into the second dialysate supply circuit 91 and the flow rate of the bypass circuit 170 is discharged to the first dialysate discharge circuit 92 through the sub-bypass circuit 200. In addition, the flow rates of the second dialysate supply circuit 91 and the first dialysate discharge circuit 92 are kept at a predetermined value or more so that a detection sensitivity of a flow rate sensor such as the flow rate sensor 111 can be maintained. Furthermore, at this time, the check valve 202 can prevent the dialysate in the first dialysate discharge circuit 92 from reversely flowing into the bypass circuit 150 by suction with the fluid replacement pump 170.

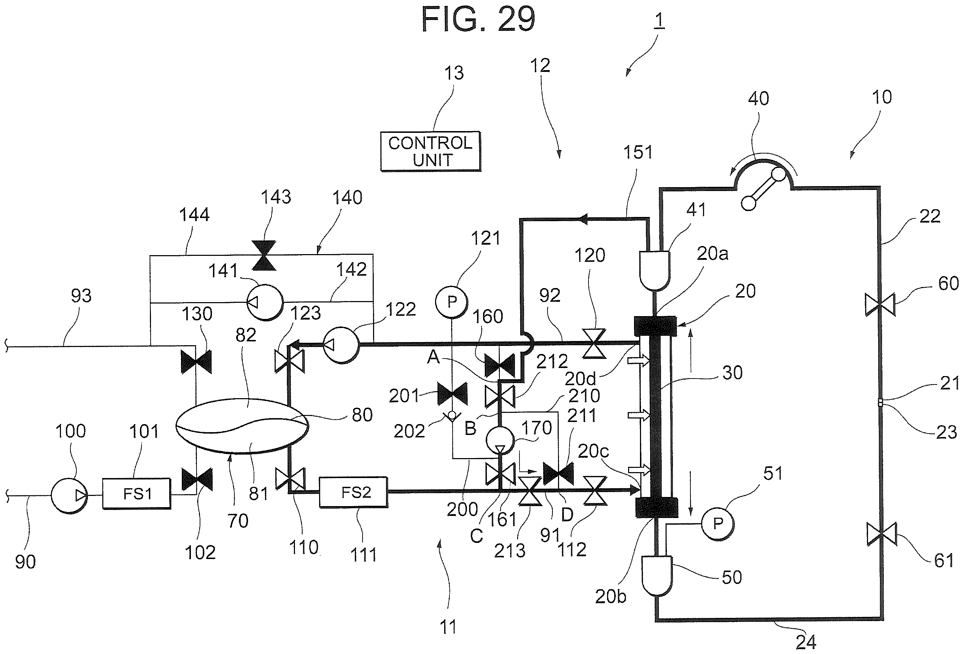

In the priming second process, as illustrated in Fig. 29, for example, the opening/closing valve 161, the opening/closing valve 212, and the opening/closing valve 213 are opened, the opening/closing valve 160, the opening/closing valve 201, and the opening/closing valve 211 are closed, and the fluid replacement pump 170 is rotated reversely so that dialysate that has flowed into the connection circuit 151 of the fluid replacement/priming circuit 12 is returned to the second dialysate supply circuit 91 through the bypass circuit 150 and supplied to the blood purification unit 20 again.

In these priming processes, a flushing operation of opening and closing the clamp valves 60 and 61 may be performed, or the flow rate of the blood pump 40 may be changed to promote removal of bubbles. The flow rate of the blood pump 40 may be higher than the flow rate of the fluid replacement pump 170, or the blood pump 40 may be rotated reversely.

In the priming processes, based on a result of pressure detection by the pressure sensor 51, the control unit 13, for example, may adjust the flow rate of the fluid replacement pump 170 so that a pressure on the blood purification membrane 30 is within a predetermined range.

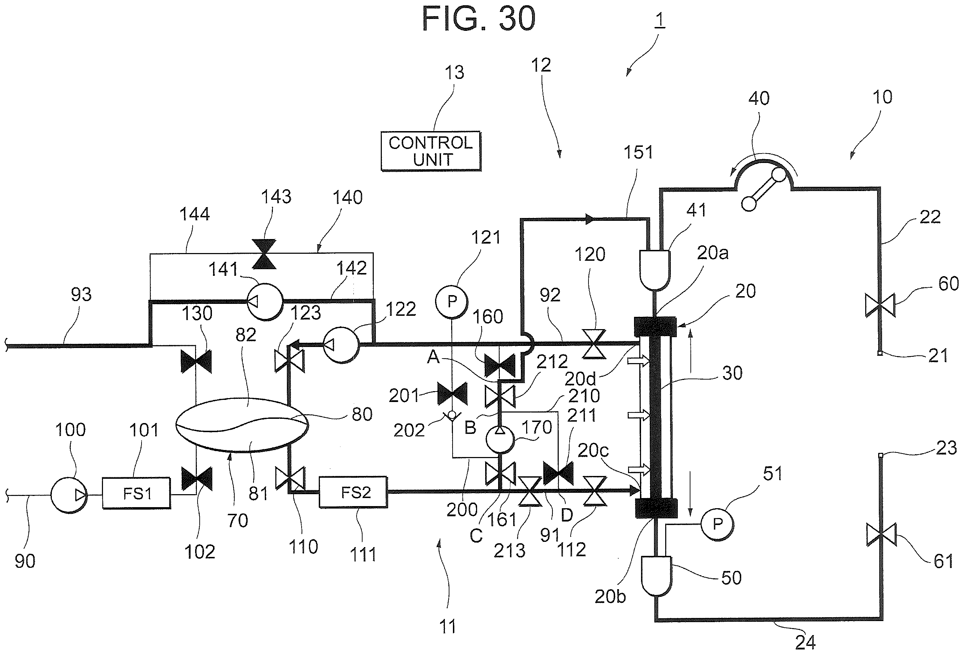

In a blood purification process, which does not form part of the invention, as illustrated in Fig. 30, the blood pump 40 rotates forwardly so that blood circulates in the blood circuit 10. In the dialysate circuit 11, dialysate is supplied from the metering pump 70 to the secondary side of the blood purification membrane 30 of the blood purification unit 20 through the second dialysate supply circuit 91, and the dialysate that has passed through the secondary side of the blood purification membrane 30 is discharged to the metering pump 70 through the first dialysate discharge circuit 92. In the blood purification unit 20, blood flows in the primary side of the blood purification membrane 30 and dialysate flows in the secondary side so that waste products in the blood are discharged to the dialysate through the blood purification membrane 30 and, thereby, the blood is purified.

In the fluid replacement/priming circuit 12, while the opening/closing valve 161 and the opening/closing valve 212 are open, the opening/closing valve 160, the opening/closing valve 201, and the opening/closing valve 211 are closed, and the fluid replacement pump 170 rotates forwardly so that the dialysate can be suitably supplied to the blood collection circuit 22 through the bypass circuit 150 and the connection circuit 151, thereby performing fluid replacement on a patient.

In this embodiment, since dialysate is supplied to the blood purification membrane 30 by using a driving force of the fluid replacement pump 170 in priming, the flow rate can be easily adjusted so that a load on the membrane can be reduced, as compared to a case where dialysate is directly supplied from the metering pump 70 to the blood purification membrane 30 through the second dialysate supply circuit 91.

In a case where the opening/closing valve 160, the opening/closing valve 161, and the opening/closing valve 212 are opened and the fluid replacement pump 170 is rotated forwardly, the dialysate in the second dialysate supply circuit 91 flows into the first dialysate discharge circuit 92 through the fluid replacement pump 170 of the bypass circuit 150. Thus, in this embodiment, it is possible to clean a channel in the fluid replacement pump 170 by using the dialysate. As a result, the fluid replacement pump 170 does not need to be discarded after each blood purification process, and can be permanently incorporated in the body of the blood purification device 1.

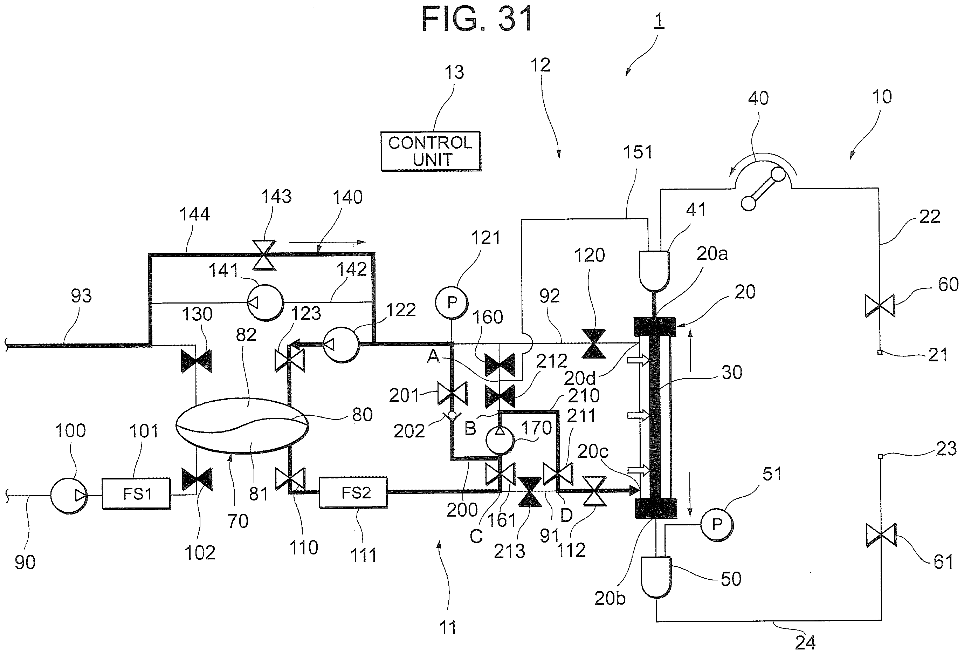

In addition, in a case where a blood pressure of the patient rapidly decreases in a blood purification process, for example, urgent fluid replacement can be performed. In this urgent fluid replacement process, which does not form part of the invention, as illustrated in Fig. 31, for example, while the opening/closing valve 161, the opening/closing valve 211, and the opening/closing valve 112 are opened, the opening/closing valve 160, the opening/closing valve 212, the opening/closing valve 213, and the opening/closing valve 120 are closed, and the fluid replacement pump 170 is rotated forwardly. In this manner, the dialysate in the second dialysate supply circuit 91 is supplied to the blood purification unit 20 through the bypass circuit 150 and the sub-circuit 210 and then supplied to the blood circuit 10 through the blood purification membrane 30 so that the dialysate is urgently supplied to the blood. This urgent fluid replacement may be performed by opening the opening/closing valve 212, closing the opening/closing valve 211, and causing dialysate to pass through the bypass circuit 150 and the connection circuit 151 to supply the dialysate to the blood circuit 10 by using the fluid replacement pump 170.

At this time, the opening/closing valve 201 and the opening/closing valve 143 of the water removing circuit 140 are also opened so that a part of the dialysate that has flowed from the second dialysate supply circuit 91 into the bypass circuit 150 and the dialysate in the second dialysate discharge circuit 93 are supplied to the dialysate discharge chamber 82 of the metering pump 70 through the first dialysate discharge circuit 92. The dialysate in the second dialysate discharge circuit 93 flows back in, for example, the second circuit 144 to be supplied to the dialysate discharge chamber 82. In this manner, a supply/discharge balance of the dialysate in the metering pump 70 in urgent fluid replacement can be obtained. At this time, the flow rates in the second dialysate supply circuit 91 and the first dialysate discharge circuit 92 are kept at a predetermined value or more so that a detection sensitivity of a flow rate sensor such as the flow rate sensor 111 can be maintained. In addition, the check valve 202 can prevent the dialysate from reversely flowing in the bypass circuit 200.

The preferred embodiments of the present invention have been described with reference to the attached drawings.

In the foregoing embodiments, the priming first process and the priming second process are performed. Alternatively, only the priming first process may be performed. The blood purification device 1 may have other circuit configurations as long as the blood purification device 1 can perform at least the priming first process. The blood purification device 1 may be applied not only to a device for performing hemodialysis but also a device for performing a continuous renal replacement therapy, for example.

### Seventh Embodiment

As described above, in the blood purification device, it is intended that dialysate in a dialysate supply circuit is supplied, as a priming solution for cleaning a circuit before a treatment or fluid for replacement to a patient under a treatment, to a blood circuit through a blood purification membrane (backfiltration process). In such a case, the dialysate might enter the body of the patient from the blood circuit, and thus, a plurality of filters for purifying the dialysate can be provided in the dialysate supply circuit connected to the blood purification unit for backup.

However, in the case where the multiple filters are provided in the dialysate supply circuit, a large pressure loss occurs in the circuit, and thus, the dialysate might not be supplied to a blood purification unit at a high flow rate in a normal dialysis treatment.

This embodiment provides a blood purification device and a priming method capable of flowing dialysate at a high flow rate while providing a plurality of filters in a dialysate supply circuit as measures against a possible single failure. Specifically, the present invention provides, for example, a blood purification device including: a blood purification unit including a blood purification membrane; a blood circuit that feeds blood in a blood collection part to a primary side of the blood purification membrane of the blood purification unit and returns the blood from the primary side of the blood purification membrane of the blood purification unit to a blood return part; a dialysate supply circuit that supplies dialysate to a secondary side of the blood purification membrane of the blood purification unit; and a dialysate discharge circuit that discharges the dialysate from the secondary side of the blood purification membrane of the blood purification unit, and the dialysate supply circuit includes a first channel having a plurality of filters and a second channel having one or more filters in a number smaller than the number of the filters in the first channel, and is configured to be switchable between the first channel and the second channel. The present invention also provides, for example, a priming method for a blood purification device including a step of, while connecting a blood collection part and a blood return part of a blood circuit to each other to form a loop shape of the blood circuit, supplying dialysate to a secondary side of a blood purification membrane of a blood purification unit through filters in a dialysate supply circuit, causing the dialysate to flow out to a primary side through the blood purification membrane, and discharging the dialysate in the blood circuit including the primary side to a dialysate discharge circuit, wherein the number of filters through which the dialysate passes in the dialysate supply circuit in the step is larger than the number of filters through which the dialysate passes in the dialysate supply circuit in a blood purification process.

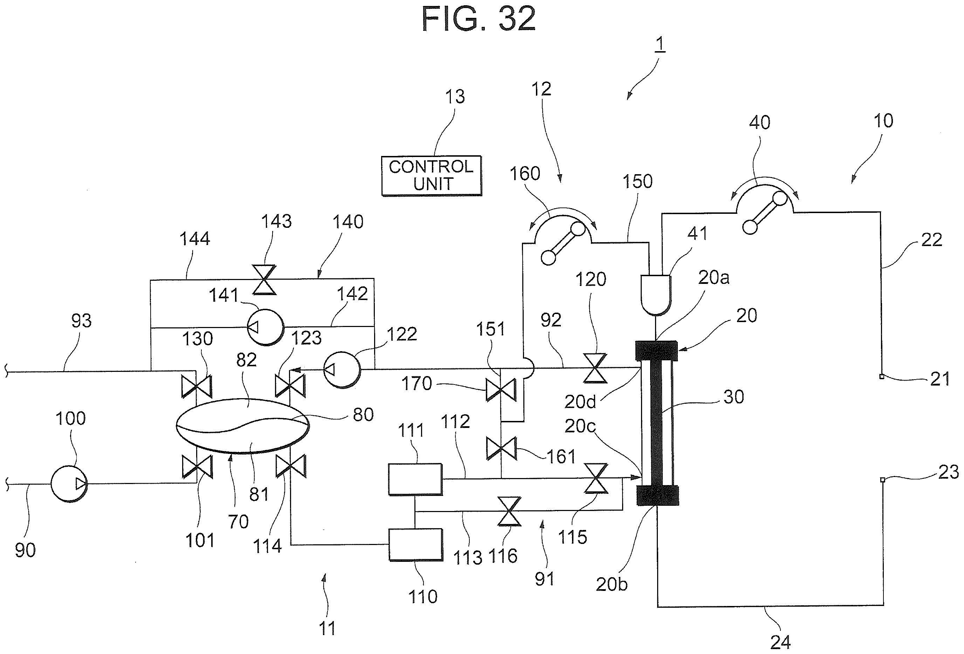

As illustrated in Fig. 32, for example, in this embodiment, the second dialysate supply circuit 91 includes a first channel 112 having a plurality of, such as two, filters 110 and 111 and a second channel 113 having a smaller number, such as one, of filters 110 than that of the first channel 112. The most upstream filter 110 is shared by the first channel 112 and the second channel 113. That is, the first channel 112 passes from the metering pump 70 through the first filter 110 and then through the second filter 111 to reach the blood purification unit 20. The second channel 113 passes from the metering pump 70 through the first filter 110 and directly reaches the blood purification unit 20.

The filters 110 and 111 are, for example, hollow fiber modules, and can remove undesired substances such as endotoxin contained in dialysate and purify the dialysate.

An opening/closing valve 114 is provided between the first filter 110 and the metering pump 70. An opening/closing valve 115 is provided between the second filter 111 and the blood purification unit 20 in the first channel 112, and an opening/closing valve 116 is provided between the first filter 110 and the blood purification unit 20 in the second channel 113. These opening/closing valves 115 and 116 constitute a switching device that can switch between the first channel 112 and the second channel 113. A downstream side of the opening/closing valve 115 of the first channel 112 and a downstream side of the opening/closing valve 116 of the second channel 113 are connected to the blood purification unit 20 through a common channel.

The fluid replacement/priming circuit 12 includes a fluid replacement circuit 150 connecting from a portion of the first channel 112 between the second filter 111 and the opening/closing valve 115 to the drip chamber 41 of the blood collection circuit 22, and a connection circuit 151 connecting the fluid replacement circuit 150 to the first dialysate discharge circuit 92. The fluid replacement circuit 150 and the connection circuit 151 are constituted by, for example, soft tubes.

The fluid replacement circuit 150 includes a fluid replacement pump 160 that is rotatable forwardly and reversely. The fluid replacement pump 160 is, for example, a tubing pump that has a flow rate ranging from about 0 to 600 mL/min and can supply about 10 mL in one turn. The fluid replacement circuit 150 includes an opening/closing valve 161.

The connection circuit 151 connects a portion of the fluid replacement circuit 150 between the fluid replacement pump 160 and the opening/closing valve 161 to the first dialysate discharge circuit 92. The connection circuit 151 includes an opening/closing valve 170. In this embodiment, the opening/closing valve 161 and the opening/closing valve 170 enable switching of connection to the first channel 112 of the fluid replacement circuit 150 and the first dialysate discharge circuit 92.

The control unit 13 is, for example, a computer for controlling an entire operation of the blood purification device 1, and can control operations of, for example, the pumps 40, 100, 122, 141, and 160 and the valves 101, 114, 115, 116, 120, 123, 130, 143, 161, and 170 and causes the blood purification device 1 to operate and perform priming and a blood purification process described later. For example, in the case of executing a program to perform a backfiltration process in which a part or the whole of the dialysate supplied to the secondary side of the blood purification membrane 30 of the blood purification unit 20 is supplied to the primary side through the blood purification membrane 30, the control unit 13, for example, controls the opening/closing valves 115 and 116 to supply the dialysate to the blood purification unit 20 through the first channel 112. On the other hand, in the case of performing no backfiltration process, the control unit 13, for example, controls the opening/closing valves 115 and 116 to switch the first channel 112 and the second channel 113 so that the dialysate is supplied to the blood purification unit 20 through the second channel 113. In this embodiment, the opening/closing valves 115 and 116 and the control unit 13, for example, constitute a switching unit.

Next, priming, which is a backfiltration process, performed in the blood purification device 1 will be described.

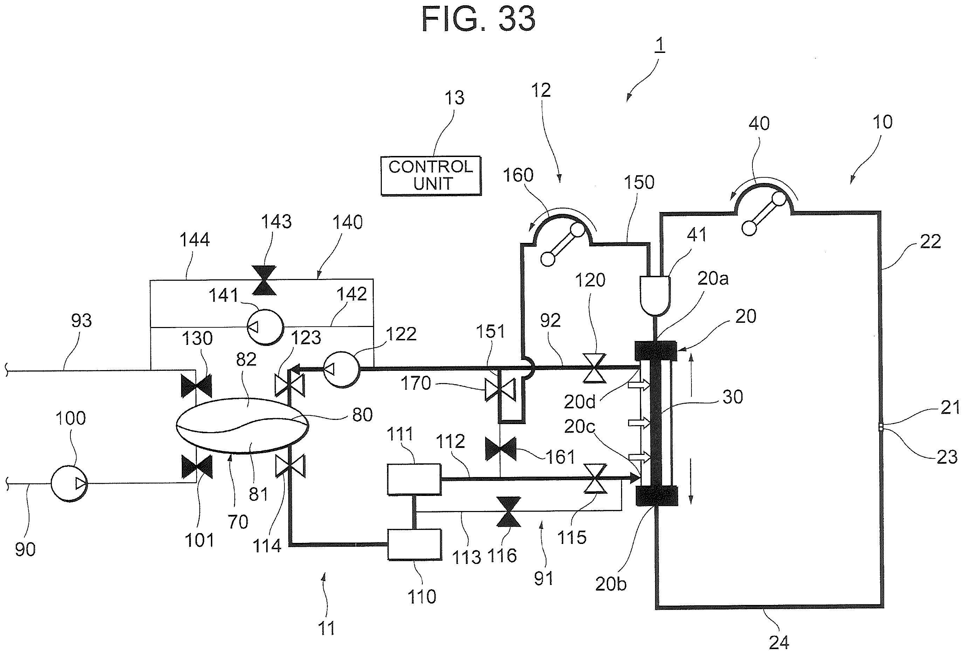

In priming, as illustrated in Fig. 33, the blood collection part 21 is connected to the blood return part 23 in the blood circuit 10, whereas the opening/closing valves 114, 115, 120, 123, and 170 are opened and the opening/closing valves 101, 116, 130, 143, and 161 are closed in the dialysate circuit 11. A fluid feed pump 122 operates, a fluid replacement pump 160 operates in a reverse rotation direction (to a direction of the first dialysate discharge circuit 92), and the blood pump 40 operates in a forward rotation direction (to a direction of the blood purification unit 20). In this manner, dialysate flowing in a dialysate supply chamber 81 of the metering pump 70 passes through the first channel 112 of the second dialysate supply circuit 91, and is supplied to the secondary side of the blood purification membrane 30 of the blood purification unit 20. That is, the dialysate is supplied to the secondary side of the blood purification membrane 30 through the two filters of the first filter 110 and the second filter 111. Part of the dialysate supplied to the secondary side of the blood purification membrane 30 is caused to flow directly into the first dialysate discharge circuit 92 by the fluid feed pump 122 and discharged to the dialysate discharge chamber 82 of the metering pump 70. The other part of the dialysate in the secondary side of the blood purification membrane 30 is caused to flow into the primary side of the blood purification membrane 30 (backfiltration process) by a negative pressure at the blood circuit 10 caused by the fluid replacement pump 160 and to be branched off by the blood pump 40 and flow in the blood circuit 10. The dialysate in the blood circuit 10 is caused to flow into the fluid replacement circuit 150 by the fluid feed pump 160, flow into the first dialysate discharge circuit 92 through the connection circuit 151, and be discharged to the dialysate discharge chamber 82 of the metering pump 70. In this manner, fluid in the blood circuit 10, the dialysate circuit 11, and the fluid replacement/priming circuit 12 is replaced and cleaned with fresh dialysate, and bubbles in these circuits are removed.

Then, a blood purification process, which does not form part of the invention, without a backfiltration process performed in the blood purification device 1 will be described.

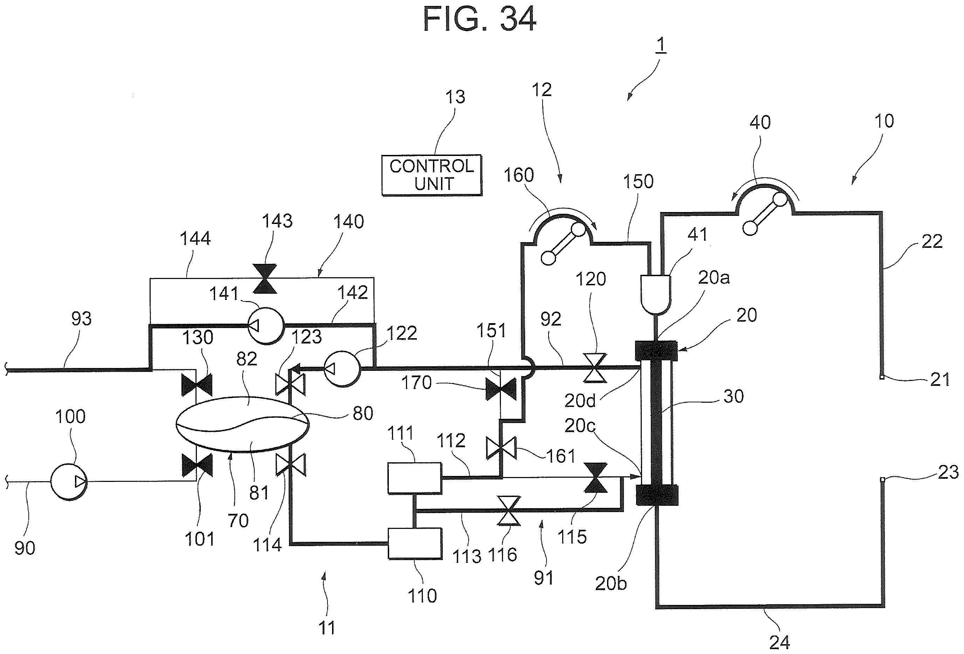

First, as illustrated in Fig. 34, an unillustrated paracentesis needle is connected to the blood collection part 21 and the blood return part 23 and put into a patient. Next, the opening/closing valves 114, 116, 120, 123, and 161 are opened and the opening/closing valves 101, 115, 130, 143, and 170 are closed in the dialysate circuit 11. The fluid feed pump 122 operates, the fluid replacement pump 160 operates in a forward rotation direction (to a direction of the blood circuit 10), and the blood pump 40 operates in a forward rotation direction (to a direction of the blood purification unit 20). In this manner, in the blood circuit 10, blood of the patient is supplied to the primary side of the blood purification membrane 30 of the blood purification unit 20 through the blood collection circuit 22, and blood that has passed through the primary side of the blood purification membrane 30 is returned to the patient through the blood return circuit 24.

In the dialysate circuit 11, the dialysate in the dialysate supply chamber 81 of metering pump 70 is supplied to the secondary side of the blood purification membrane 30 of the blood purification unit 20 through the second channel 113 of the second dialysate supply circuit 91. That is, the dialysate in the second dialysate supply circuit 91 is supplied to the secondary side of the blood purification membrane 30 only through the first filter 110. The dialysate that has passed through the secondary side of the blood purification membrane 30 is discharged to the dialysate discharge chamber 82 of the metering pump 70 through the first dialysate discharge circuit 92. In the metering pump 70, when the partition 80 moves and the dialysate is completely consumed in the dialysate supply chamber 81, the opening/closing valves 101 and 130 are opened, the opening/closing valves 114 and 123 are closed, and the dialysate supply pump 100 supplies fresh dialysate to the dialysate supply chamber 81 through the first dialysate supply circuit 90. At this time, the dialysate in the dialysate discharge chamber 82 is discharged through the second dialysate discharge circuit 93.

In this manner, in the blood purification unit 20, blood flows in the primary side of the blood purification membrane 30 and dialysate flows in the secondary side so that waste products in the blood are discharged to the dialysate through the blood purification membrane 30 and, thereby, the blood is purified.

Part of dialysate in the metering pump 70 flows into the first channel 112, passes through the first filter 110 and the second filter 111, and is supplied to the blood circuit 10 through the fluid replacement circuit 150 by the fluid replacement pump 160. In this manner, fluid replacement is performed on the patient.

In the water removing circuit 140, the water removing pump 141 operates and the opening/closing valve 143 is closed so that part of dialysate in the first dialysate discharge circuit 92 is discharged to the second dialysate discharge circuit 93 through the water removing circuit 140 without passing through the metering pump 70. The amount of dialysate passing through the water removing circuit 140 at this time is adjusted in accordance with the amount of water removed from the blood of the patient.

In this embodiment, the second dialysate supply circuit 91 includes the first channel 112 having the two filters 110 and 111 and the second channel 113 having one or more filters 110 in a number smaller than the number of filters of the first channel 112, and can switch between the first channel 112 and the second channel 113. Thus, in a case where dialysate is supplied through the blood purification membrane 30 of the blood purification unit 20 to the blood circuit 10 where the dialysate might enter the body of a patient, the first channel 112 having a plurality of filters for backup is used to flow the dialysate. Thus, in a case where the dialysate is not supplied to the blood circuit 10 through the blood purification membrane 30, the dialysate can flow using the second channel 113 having a small number of filters and showing a small pressure loss. Thus, the dialysate can flow at a high flow rate while a plurality of filters 110 and 111 are disposed in the second dialysate supply circuit 91 as measures against a possible single failure.

In this embodiment, the blood purification device 1 includes the switching unit that performs switching between the first channel 112 and the second channel 113 in such a manner that in the case of priming, which is a backfiltration process in which a part or the whole of the dialysate supplied to the secondary side of the blood purification membrane 30 of the blood purification unit 20 is supplied to the primary side through the blood purification membrane 30, the dialysate is supplied to the blood purification unit 20 through the first channel 112, whereas in the case of a dialysis process in which a backfiltration process is not performed, the dialysate is supplied to the blood purification unit 20 through the second channel 113. Thus, switching between the first channel 112 and the second channel 113 can be suitably performed between the backfiltration process that needs backup of filters and the process without backfiltration that does not need backup of filters.

The blood purification device 1 includes the fluid replacement circuit 150 connecting downstream sides of the filters 110 and 111 in the first channel 112 to the blood circuit 10, and the second dialysate supply circuit 91 is configured to supply dialysate to the fluid replacement circuit 150 through the first channel 112. Thus, the dialysate to be supplied as a replacement to the blood circuit 10 without passing through the blood purification membrane 30 is allowed to pass through the filters 110 and 111. Thus, a circuit configured as measures against a single failure can also be used for replacement of fluid supplied to the blood circuit 10 without passing through the blood purification membrane 30.

The blood purification device 1 includes the connection circuit 151 connecting the fluid replacement circuit 150 to the first dialysate discharge circuit 92, and is configured to be switchable between connection to the first channel 112 of the fluid replacement circuit 150 and connection to the first dialysate discharge circuit 92. Thus, the dialysate in the blood circuit 10 can be discharged to the first dialysate discharge circuit 92 through the fluid replacement circuit 150. Thus, priming using backfiltration can be suitably performed.

Since the first channel 112 and the second channel 113 share the first filter 110, even in the presence of the two channels 112 and 113 in the second dialysate supply circuit 91, an increase in the number of filters can be suppressed.

In addition, since the common filter 110 is disposed upstream of other filters 111, dialysate can flow in, for example, the first channel 112 and the second channel 113 at the same time and parts of the dialysate flowing in these channels are allowed to flow into different circuits. That is, in a manner similar to that in this embodiment, in a dialysis process, which does not form part of the invention, while dialysate that has passed through one filter is supplied to the blood purification unit 20, dialysate that has passed through two filters is allowed to be supplied to the blood circuit 10 for replacement.

In priming in this embodiment, the number (two) of filters through which dialysate passes in the second dialysate supply circuit 91 is larger than the number (one) of filters through which dialysate passes in the second dialysate supply circuit 91 in a blood purification process. Thus, a high flow rate of dialysate supplied to the blood circuit 10 in priming, which is a backfiltration process, can be achieved with a small number of filters in the blood purification process while taking measures against a single failure of a filter.

In the blood purification device 1 described in the embodiment above, in a case where the flow rate of dialysate supplied to the most upstream filter is less than 700 mL/min, the dialysate is also distributed to the first channel 112, whereas in a case where this flow rate is 700 mL/min or higher, the dialysate is distributed to the second channel 113.

In the blood purification process, which does not form part of the invention, for example, in a case where the flow rate of dialysate supplied to the first filter 110 is 700 mL/min or higher, the fluid feed pump 122 and the fluid replacement pump 160 operate with the opening/closing valves 114, 116, 120, 123, and 161 being open and the opening/closing valves 115 and 170 being closed in the dialysate circuit 11, as illustrated in Fig. 34. In this manner, dialysate flowing in the dialysate supply chamber 81 of metering pump 70 is supplied to the blood purification unit 20 through the second channel 113 of the second dialysate supply circuit 91. Part of the dialysate is supplied to the blood circuit 10 through the fluid replacement circuit 150 for replacement. In this example, since dialysate supplied to the blood purification unit 20 flows in the second channel 113, a desired flow rate of dialysate can be obtained without a decrease in the flow rate.

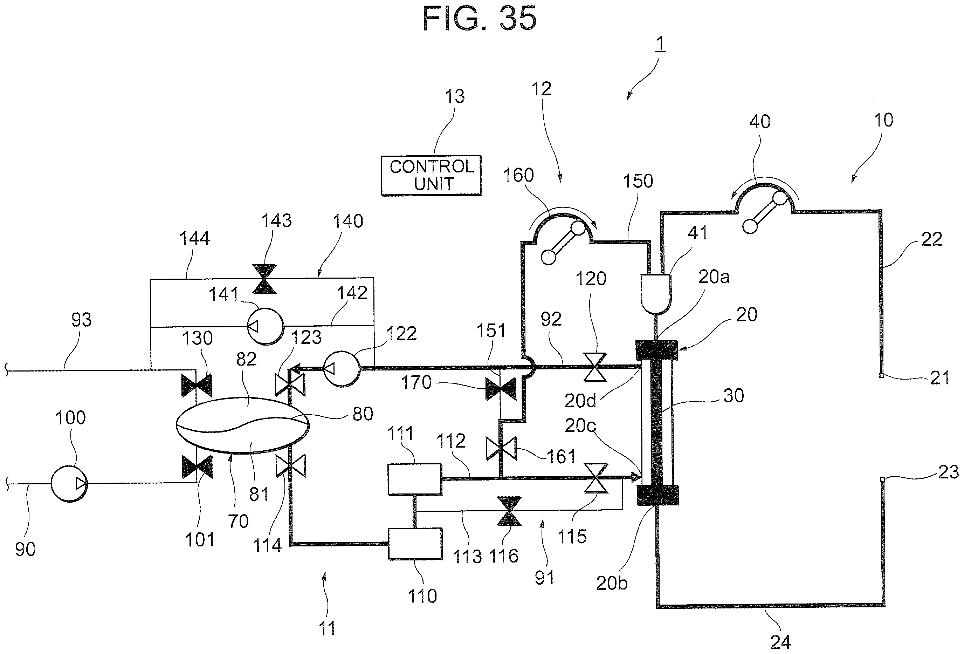

On the other hand, as illustrated in Fig. 35, in a case where the flow rate of dialysate supplied to the first filter 110 is less than 700 mL/min, the fluid feed pump 122 and the fluid replacement pump 160 operate with the opening/closing valves 114, 115, 120, 123, and 161 being open and the opening/closing valves 116 and 170 being closed in the dialysate circuit 11. In this manner, dialysate in the dialysate supply chamber 81 of metering pump 70 is supplied to the blood purification unit 20 through the first channel 112 of the second dialysate supply circuit 91. Part of the dialysate is supplied to the blood circuit 10 through the fluid replacement circuit 150 for replacement. In this examples, dialysate that is not required of a high flow rate flows in the first channel 112, and thus, dialysate can flow in a channel for backup of filters.

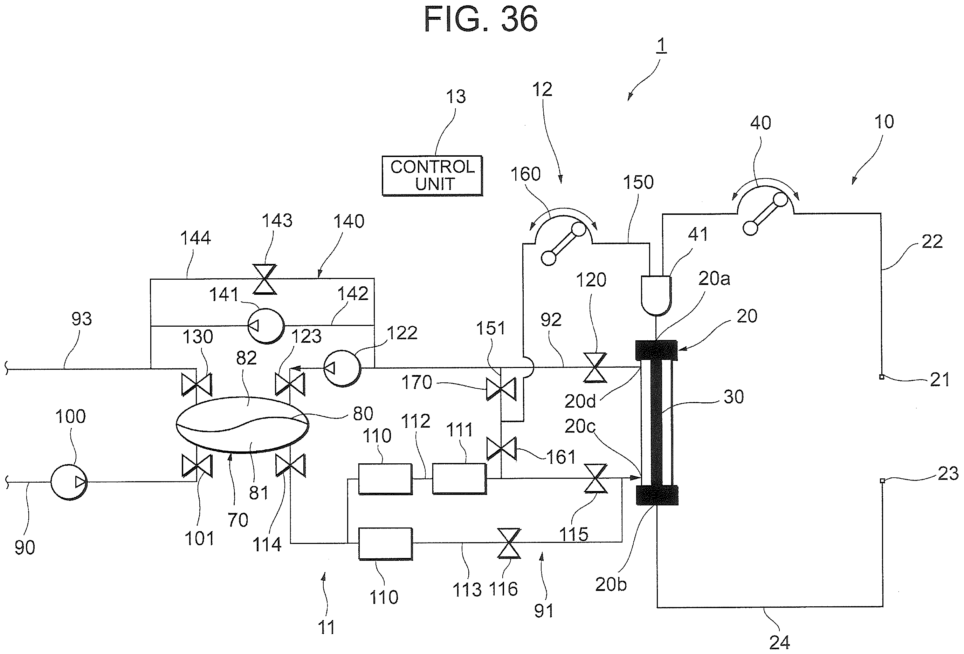

In this embodiment, the number of filters in the first channel 112 is two and the number of filter in the second channel 113 is one. Alternatively, these numbers of filters may be freely selected as long as the number of filters in the first channel is larger than the number of filters in the second channel. In the embodiment, the first channel and the second channel share the first filter. Alternatively, as illustrated in Fig. 36, the second dialysate supply circuit 91 may include parallel first and second channels 112 and 113 that do not share a filter.

In this embodiment, the backfiltration process is performed in priming. Alternatively, the backfiltration process may be performed in another process in which dialysate is supplied to the blood purification unit 20 through the first channel 112. In this process in which the backfiltration process is performed, fluid replacement maybe performed from the second dialysate supply circuit 91 to the blood circuit 10 through the blood purification membrane 30, for example. In the present invention, even in a case where the backfiltration process is not performed, dialysate may be supplied to the blood purification unit 20 using the first channel 112.

The circuit configuration and device configuration of the blood purification device 1 according to this embodiment are not limited to those described in this embodiment, and another circuit configuration and another device configuration may be employed. The blood purification device 1 may be applied not only to a device for performing hemodialysis but also to a device for performing a continuous renal replacement therapy, for example.

In addition, in this embodiment, the blood purification device includes a fluid replacement/priming circuit configured to perform: a fluid replacement process that does not form part of the invention in which dialysate in the dialysate supply circuit is supplied to the blood circuit by using the fluid replacement pump; and a priming process in which, while a blood collection part and a blood return part is connected to each other to form a loop of the blood circuit, dialysate in the dialysate supply circuit is caused to flow out to the primary side through the blood purification membrane by using the fluid replacement pump and dialysate in the blood circuit including the primary side is discharged to the dialysate discharge circuit. However, the present invention is also applicable to a blood purification circuit not including such a fluid replacement/priming circuit.

### Industrial Applicability

The present invention is useful in suitably performing priming and fluid replacement of a blood purification device by using a pump having a flow rate and a flow rate accuracy suitable for priming.

### Reference Signs List

- 1: blood purification device
- 10: blood circuit
- 11: dialysate circuit
- 12: fluid replacement/priming circuit
- 13: control unit
- 20: blood purification unit
- 30: blood purification membrane
- 40: blood pump
- 70: metering pump
- 91: second dialysate supply circuit
- 92: first dialysate discharge circuit
- 141: water removing pump

## Claims

1. A blood purification device (1) comprising:
a blood purification unit (20) including a blood purification membrane (30);
a blood circuit (10) that feeds blood in a blood collection part (21) to a primary side of the blood purification membrane (30) of the blood purification unit (20) and returns the blood from the primary side of the blood purification membrane (30) of the blood purification unit (20) to a blood return part (23), by using a blood pump (40);
a dialysate supply circuit (90) that supplies dialysate to a secondary side of the blood purification membrane (30) of the blood purification unit (20);
a dialysate discharge circuit (92) that discharges the dialysate from the secondary side of the blood purification membrane (30) of the blood purification unit (20); and
a fluid replacement/priming circuit (12) configured to perform
a fluid replacement process in which the dialysate in the dialysate supply circuit (90) is supplied to the blood circuit (10) by using a fluid replacement pump (170) and
a priming process in which while the blood collection part (21) and the blood return part (23) are connected to each other to form a loop shape of the blood circuit (10), the dialysate in the dialysate supply circuit (90) is caused to flow out to the primary side through the blood purification membrane (30) and the dialysate in the blood circuit (10) including the primary side is discharged to the dialysate discharge circuit (92) by using the fluid replacement pump (170), wherein
the fluid replacement/priming circuit (12) is connected to the blood circuit (10), and is configured to be selectively connectable to the dialysate supply circuit (90) and the dialysate discharge circuit (92), and
the fluid replacement/priming circuit (12) includes a bypass circuit (150) connecting the dialysate supply circuit (90) to the dialysate discharge circuit (92), and a connection circuit (151) connecting the blood circuit (10) to an intermediate portion of the bypass circuit (150),
the bypass circuit (150) includes a first flow rate adjusting unit (160) disposed between a connection point (A) to the connection circuit (151) and a connection point to the dialysate discharge circuit (92) and configured to be freely opened and closed, and a second flow rate adjusting unit (161) disposed between a connection point (A) to the connection circuit (151) and a connection point to the dialysate supply circuit (90) and configured to be freely opened and closed, **characterized in that**
the fluid replacement pump (170) is rotatable forwardly and reversely and is disposed in the connection circuit (151).

2. The blood purification device (1) according to claim 1, wherein
the fluid replacement/priming circuit (12) is configured to perform a priming second process in which while the blood collection part (21) and the blood return part (23) are connected to each other to form a loop shape of the blood circuit (10), the dialysate in the dialysate supply circuit (90) is caused to flow out to the primary side through the blood purification membrane (30) and the dialysate in the blood circuit (10) including the primary side is returned to the dialysate supply circuit (90) by using the fluid replacement pump (170).

3. The blood purification device (1) according to any one of claims 1 or 2, wherein
the blood circuit (10) includes a blood collection circuit (22) connecting the blood collection part (21) to the blood purification unit (20) and a blood return circuit (24) connecting the blood purification unit (20) to the blood return part (23), and
the connection circuit (151) is configured to be connectable to at least one of the blood collection circuit (22) or the blood return circuit (24).

4. The blood purification device (1) according to any one of claims 1 to 3, further comprising a blood returning circuit (24) connecting a portion of the blood circuit (10) closer to the blood collection part (21) than the blood pump (40) to the connection circuit (151).

5. The blood purification device (1) according to any one of claims 1 to 4, further comprising:
a pressure sensor (51) configured to detect a pressure of at least one of the primary side or the secondary side of the blood purification membrane (30); and
a control unit (13) that adjusts a flow rate of the fluid replacement pump (170) so that a pressure on the blood purification membrane (30) is within a predetermined range, based on a result of detection by the pressure sensor (51).

6. The blood purification device (1) according to any one of claims 1 to 5, further comprising a control unit (13) that adjusts a flow rate of the blood pump (40) so that the flow rate of the blood pump (40) is lower than a flow rate of the fluid replacement pump (170) in the priming process.

7. The blood purification device (1) according to any one of claims 1 or 2, wherein
the dialysate supply circuit (90) includes a first channel (112) having a plurality of filters (110, 111) and a second channel (113) having one or more filters (110, 111) in a number smaller than the number of the filters in the first channel (112), and is configured to be switchable between the first channel (112) and the second channel (113).

8. The blood purification device (1) according to claim 7, further comprising a switching unit that performs switching between the first channel (112) and the second channel (113) in such a manner that in the case of performing a backfiltration process in which a part or whole of the dialysate supplied to the secondary side of the blood purification membrane (30) of the blood purification unit (20) is supplied to the primary side through the blood purification membrane (30), the dialysate is supplied to the blood purification unit (20) through the first channel (112), whereas in the case of not performing the backfiltration process, the dialysate is supplied to the blood purification unit (20) through the second channel (113).

9. The blood purification device (1) according to claim 7 or 8, further comprising
a fluid replacement circuit (150) connecting a downstream side of the plurality of filters (110, 111) in the first channel (112) to the blood circuit (10), wherein
the dialysate supply circuit (90) is configured to supply dialysate to the fluid replacement circuit (150) through the first channel (112).

10. The blood purification device (1) according to claim 9, further comprising
a connection circuit (151) connecting the fluid replacement circuit (150) to the dialysate discharge circuit (92), wherein
the blood purification device (1) is configured to be switchable between connection to the first channel (112) of the fluid replacement circuit (150) and connection to the dialysate discharge circuit (92).

11. The blood purification device (1) according to any one of claims 7 to 10, wherein the first channel (112) and the second channel (113) share one or more of the filters (110, 111).

12. The blood purification device (1) according to claim 11, wherein
the one or more filters (110, 111) shared by the first and second channels (112, 113) are disposed upstream of the other filters.

13. The blood purification device (1) according to any one of claims 7 to 12, wherein
in a case where the flow rate of dialysate supplied to a most upstream filter is less than 700 mL/min, the dialysate is distributed to the first channel (112), whereas in a case where the flow rate is 700 mL/min or higher, the dialysate is distributed to the second channel (113).

14. A method for priming of the blood purification device (1) as defined in claim 1, the method comprising
a step of, while connecting a blood collection part (21) and a blood return part (23) of a blood circuit (10) to each other to form a loop shape of the blood circuit (10), supplying dialysate to a secondary side of a blood purification membrane (30) of a blood purification unit (20) through filters in a dialysate supply circuit (90), causing the dialysate to flow out to a primary side through the blood purification membrane (30), and discharging the dialysate in the blood circuit (10) including the primary side to a dialysate discharge circuit (92), wherein the number of filters (110, 111) through which the dialysate passes in the dialysate supply circuit (90) in the step is larger than the number of filters through which the dialysate passes in the dialysate supply circuit (90) in a blood purification process.

## Patentansprüche

1. Blutreinigungsvorrichtung (1), umfassend:
eine Blutreinigungseinheit (20), die eine Blutreinigungsmembran (30) umfasst;
einen Blutkreislauf (10), der Blut in einem Blutsammelteil (21) einer primären Seite der Blutreinigungsmembran (30) der Blutreinigungseinheit (20) zuführt und das Blut von der primären Seite der Blutreinigungsmembran (30) der Blutreinigungseinheit (20) aus durch Verwendung einer Blutpumpe (40) zu einem Blutrückführungsteil (23) zurückführt;
einen Dialysatzuführungskreislauf (90), der Dialysat einer sekundären Seite der Blutreinigungsmembran (30) der Blutreinigungseinheit (20) zuführt;
einen Dialysat-Ausleitungskreislauf (92), der das Dialysat aus der sekundären Seite der Blutreinigungsmembran (30) der Blutreinigungseinheit (20) ausleitet; und
einen Flüssigkeitsersatz-/-vorbehandlungskreislauf (12), der so konfiguriert ist, dass er Folgendes durchführt:
einen Flüssigkeitsersatzvorgang, bei dem das Dialysat in dem Dialysatzuführungskreislauf (90) durch Verwendung einer Flüssigkeitsersatzpumpe (170) dem Blutkreislauf (10) zugeführt wird; und
einen Vorbehandlungsvorgang, bei dem, während der Blutsammelteil (21) und der Blutrückführungsteil (23) unter Bildung einer Schleifenform des Blutkreislaufs (10) miteinander verbunden sind, bewirkt wird, dass das Dialysat im Dialysatzuführungskreislauf (90) durch die Blutreinigungsmembran (30) hindurch zur primären Seite hin ausströmt und das Dialysat im Blutkreislauf (10) einschließlich der primären Seite durch Verwendung der Flüssigkeitsersatzpumpe (170) zum Dialysat-Ausleitungskreislauf (92) ausgeleitet wird,
wobei der Flüssigkeitsersatz-/-vorbehandlungskreislauf (12) mit dem Blutkreislauf (10) verbunden ist und so konfiguriert ist, dass er selektiv mit dem Dialysatzuführungskreislauf (90) und dem Dialysat-Ausleitungskreislauf (92) verbunden werden kann; und
der Flüssigkeitsersatz-/-vorbehandlungskreislauf (12) einen Bypass-Kreislauf (150), der den Dialysatzuführungskreislauf (90) mit dem Dialysat-Ausleitungskreislauf (92) verbindet, und einen Verbindungskreislauf (151), der den Blutkreislauf (10) mit einem Zwischenteil des Bypass-Kreislaufs (150) verbindet, umfasst;
der Bypass-Kreislauf (150) eine erste Strömungsgeschwindigkeitseinstellungseinheit (160), die sich zwischen einem Verbindungspunkt (A) zum Verbindungskreislauf (151) und einem Verbindungspunkt zum Dialysat-Ausleitungskreislauf (92) befindet und so konfiguriert ist, dass er sich frei öffnen und schließen lässt, und eine zweite Strömungsgeschwindigkeitseinstellungseinheit (161), die sich zwischen einem Verbindungspunkt (A) zum Verbindungskreislauf (151) und einem Verbindungspunkt zum Dialysat-Zuführungskreislauf (90) befindet und so konfiguriert ist, dass er sich frei öffnen und schließen lässt, umfasst;
**dadurch gekennzeichnet, dass** die Flüssigkeitsersatzpumpe (170) vorwärts und rückwärts drehbar ist und sich im Verbindungskreislauf (151) befindet.

2. Blutreinigungsvorrichtung (1) gemäß Anspruch 1, wobei
der Flüssigkeitsersatz-/-vorbehandlungskreislauf (12) so konfiguriert ist, dass er einen zweiten Vorbehandlungsvorgang durchführt, bei dem, während der Blutsammelteil (21) und der Blutrückführungsteil (23) unter Bildung einer Schleifenform des Blutkreislaufs (10) miteinander verbunden sind, bewirkt wird, dass das Dialysat im Dialysatzuführungskreislauf (90) durch die Blutreinigungsmembran (30) hindurch zur primären Seite hin ausströmt und das Dialysat im Blutkreislauf (10) einschließlich der primären Seite durch Verwendung der Flüssigkeitsersatzpumpe (170) zum Dialysatzuführungskreislauf (90) ausgeleitet wird.

3. Blutreinigungsvorrichtung (1) gemäß einem der Ansprüche 1 oder 2, wobei
der Blutkreislauf (10) einen Blutsammelkreislauf (22), der den Blutsammelteil (21) mit der Blutreinigungseinheit (20) verbindet, und einen Blutrückführungskreislauf (24), der die Blutreinigungseinheit (20) mit dem Blutrückführungsteil (23) verbindet, umfasst; und
der Verbindungskreislauf (151) so konfiguriert ist, dass er mit dem Blutsammelkreislauf (22) und/oder dem Blutrückführungskreislauf (24) verbunden werden kann.

4. Blutreinigungsvorrichtung (1) gemäß einem der Ansprüche 1 bis 3, weiterhin umfassend einen Blutrückführungskreislauf (24), der einen Teil des Blutkreislaufs (10), der näher bei dem Blutsammelteil (21) liegt als die Blutpumpe (40), mit dem Verbindungskreislauf (151) verbindet.

5. Blutreinigungsvorrichtung (1) gemäß einem der Ansprüche 1 bis 4, weiterhin umfassend:
einen Drucksensor (51), der so konfiguriert ist, dass er einen Druck auf der primären Seite und/oder der sekundären Seite der Blutreinigungsmembran (30) nachweist; und
eine Steuerungseinheit (13), die die Strömungsgeschwindigkeit der Flüssigkeitsersatzpumpe (170) auf der Grundlage eines Nachweisergebnisses des Drucksensors (51) so einstellt, dass der Druck auf der Blutreinigungsmembran (30) in einem vorbestimmten Bereich liegt.

6. Blutreinigungsvorrichtung (1) gemäß einem der Ansprüche 1 bis 5, weiterhin umfassend eine Steuerungseinheit (13), die die Strömungsgeschwindigkeit der Blutpumpe (40) so einstellt, dass die Strömungsgeschwindigkeit der Blutpumpe (40) im Vorbehandlungsvorgang geringer ist als die Strömungsgeschwindigkeit der Flüssigkeitsersatzpumpe (170).

7. Blutreinigungsvorrichtung (1) gemäß einem der Ansprüche 1 oder 2, wobei
der Dialysatzuführungskreislauf (90) einen ersten Kanal (112), der eine Vielzahl von Filtern (110, 111) aufweist, und einen zweiten Kanal (113), der ein oder mehrere Filter (110, 111) in einer kleineren Anzahl als der erste Kanal (112) aufweist, umfasst und so konfiguriert ist, dass er zwischen dem ersten Kanal (112) und dem zweiten Kanal (113) hin- und hergeschaltet werden kann.

8. Blutreinigungsvorrichtung (1) gemäß Anspruch 7, weiterhin umfassend eine Schalteinheit, die das Umschalten zwischen dem ersten Kanal (112) und dem zweiten Kanal (113) so durchführt, dass im Falle der Durchführung eines Rückfiltrationsvorgangs, bei dem ein Teil oder das gesamte Dialysat, das der sekundären Seite der Blutreinigungsmembran (30) der Blutreinigungseinheit (20) zugeführt wird, durch die Blutreinigungsmembran (30) hindurch der primären Seite zugeführt wird, das Dialysat durch den ersten Kanal (112) hindurch der Blutreinigungseinheit (20) zugeführt wird, während im Falle, dass der Rückfiltrationsvorgang nicht durchgeführt wird, das Dialysat durch den zweiten Kanal (113) hindurch der Blutreinigungseinheit (20) zugeführt wird.

9. Blutreinigungsvorrichtung (1) gemäß Anspruch 7 oder 8, weiterhin umfassend
einen Flüssigkeitsersatzkreislauf (150), der die Stromabwärtsseite der Vielzahl von Filtern (110, 111) im ersten Kanal (112) mit dem Blutkreislauf (10) verbindet, wobei
der Dialysatzuführungskreislauf (90) so konfiguriert ist, dass er Dialysat durch den ersten Kanal (112) dem Flüssigkeitsersatzkreislauf (150) zuführt.

10. Blutreinigungsvorrichtung (1) gemäß Anspruch 9, weiterhin umfassend
einen Verbindungskreislauf (151), der den Flüssigkeitsersatzkreislauf (150) mit dem Dialysat-Ausleitungskreislauf (92) verbindet, wobei
die Blutreinigungsvorrichtung (1) so konfiguriert ist, dass sie zwischen einer Verbindung zum ersten Kanal (112) des Flüssigkeitsersatzkreislaufs (150) und einer Verbindung zum Dialysat-Ausleitungskreislauf (92) hin- und hergeschaltet werden kann.

11. Blutreinigungsvorrichtung (1) gemäß einem der Ansprüche 7 bis 10, wobei der erste Kanal (112) und der zweite Kanal (113) einen oder mehrere der Filter (110, 111) gemeinsam haben.

12. Blutreinigungsvorrichtung (1) gemäß Anspruch 11, wobei sich der eine oder die mehreren Filter (110, 111), die der erste und der zweite Kanal (112, 113) gemeinsam haben, stromaufwärts der anderen Filter befinden.

13. Blutreinigungsvorrichtung (1) gemäß einem der Ansprüche 7 bis 12, wobei
in einem Fall, bei dem die Strömungsgeschwindigkeit des dem am weitesten stromaufwärts gelegenen Filter zugeführten Dialysats kleiner als 700 ml/min ist, das Dialysat in den ersten Kanal (112) geleitet wird, während in einem Fall, bei dem die Strömungsgeschwindigkeit 700 ml/min oder mehr beträgt, das Dialysat in den zweiten Kanal (113) geleitet wird.

14. Verfahren zur Vorbereitung der Blutreinigungsvorrichtung (1) gemäß Anspruch 1, wobei das Verfahren umfasst:
einen Schritt, bei dem, während der Blutsammelteil (21) und der Blutrückführungsteil (23) eines Blutkreislaufs (10) unter Bildung einer Schleifenform des Blutkreislaufs (10) miteinander verbunden werden, Dialysat einer sekundären Seite einer Blutreinigungsmembran (30) über Filter in einem Dialysatzuführungskreislauf (90) einer Blutreinigungseinheit (20) zugeführt wird, was bewirkt, dass das Dialysat durch die Blutreinigungsmembran (30) hindurch zu einer primären Seite hin ausströmt und das Dialysat im Blutkreislauf (10) einschließlich der primären Seite zu einem Dialysat-Ausleitungskreislauf (92) ausgeleitet wird, wobei die Anzahl von Filtern (110, 111), durch die das Dialysat im Dialysatzuführungskreislauf (90) in dem Schritt hindurchtritt, größer ist als die Anzahl von Filtern, durch die das Dialysat in einem Blutreinigungsverfahren in dem Dialysatzuführungskreislauf (90) hindurchtritt.

## Revendications

1. Dispositif de purification du sang (1) comprenant :
une unité de purification du sang (20) comportant une membrane de purification du sang (30) ;
un circuit sanguin (10) qui amène le sang d'une partie de collecte du sang (21) vers un côté principal de la membrane de purification du sang (30) de l'unité de purification du sang (20) et ramène le sang depuis le côté principal de la membrane de purification du sang (30) de l'unité de purification du sang (20) vers une partie de retour du sang (23), en utilisant une pompe à sang (40) ;
un circuit d'approvisionnement en dialysat (90), qui alimente en dialysat un côté secondaire de la membrane de purification du sang (30) de l'unité de purification du sang (20) ;
un circuit de renvoi du dialysat (92) qui renvoie le dialysat depuis le côté secondaire de la membrane de purification du sang (30) de l'unité de purification du sang (20) ; et
un circuit de remplacement/amorçage du fluide (12) configuré pour réaliser
un processus de remplacement de fluide dans lequel le dialysat du circuit d'approvisionnement en dialysat (90) est amené au circuit sanguin (10) en utilisant une pompe de remplacement de fluide (170) et
un processus d'amorçage dans lequel, lorsque la partie de collecte du sang (21) et la partie de retour du sang (23) sont connectées l'une à l'autre pour former une forme de boucle du circuit sanguin (10), le dialysat présent dans le circuit d'approvisionnement en dialysat (90) est amené à s'écouler vers le côté principal à travers la membrane de purification du sang (30) et le dialysat présent dans le circuit sanguin (10) comportant le côté primaire est renvoyé vers le circuit de renvoi du dialysat (92) en utilisant la pompe de remplacement de fluide (170), où
le circuit de remplacement/amorçage du fluide (12) est connecté au circuit sanguin (10) et est configuré pour pouvoir être connecté sélectivement au circuit d'approvisionnement en dialysat (90) et au circuit de renvoi du dialysat (92), et
le circuit de remplacement/amorçage du fluide (12) comporte un circuit de dérivation (150) connectant le circuit d'approvisionnement en dialysat (90) au circuit de renvoi du dialysat (92), et un circuit de connexion (151) connectant le circuit sanguin (10) à une portion intermédiaire du circuit de dérivation (150),
le circuit de dérivation (150) comporte une première unité d'ajustement du débit (160) disposée entre un point de connexion (A) au circuit de connexion (151) et un point de connexion au circuit de renvoi du dialysat (92) et configurée pour être librement ouverte et fermée, et une seconde unité d'ajustement du débit (161) disposée entre un point de connexion (A) au circuit de connexion (151) et un point de connexion au circuit d'approvisionnement en dialysat (90) et configurée pour être librement ouverte et fermée, **caractérisé en ce que**
la pompe de remplacement de fluide (170) peut tourner en avant et en arrière, et est disposée dans le circuit de connexion (151).

2. Dispositif de purification du sang (1) selon la revendication 1, dans lequel
le circuit de remplacement/amorçage du fluide (12) est configuré pour réaliser un second processus d'amorçage dans lequel, lorsque la partie de collecte du sang (21) et la partie de retour du sang (23) sont connectées l'une à l'autre pour former une forme de boucle du circuit sanguin (10), le dialysat présent dans le circuit d'approvisionnement en dialysat (90) est amené à s'écouler vers le côté principal à travers la membrane de purification du sang (30) et le dialysat présent dans le circuit sanguin (10) comportant le côté principal, est renvoyé vers le circuit d'alimentation en dialysat (90) en utilisant la pompe de remplacement de fluide (170).

3. Dispositif de purification du sang (1) selon l'une quelconque des revendications 1 ou 2, dans lequel
le circuit sanguin (10) comporte un circuit de collecte de sang (22) connectant la partie de collecte du sang (21) à l'unité de purification du sang (20) et un circuit de retour de sang (24) connectant l'unité de purification du sang (20) à la partie retour du sang (23), et
le circuit de connexion (151) est configuré pour pouvoir être connecté à au moins un circuit parmi le circuit de collecte de sang (22) et le circuit de retour de sang (24).

4. Dispositif de purification du sang (1) selon l'une quelconque des revendications 1 à 3, comprenant en outre un circuit de retour de sang (24) connectant une portion du circuit sanguin (10) plus proche de la partie de collecte du sang (21) que la pompe à sang (40) au circuit de connexion (151).

5. Dispositif de purification du sang (1) selon l'une quelconque des revendications 1 à 4, comprenant en outre :
un capteur de pression (51) configuré pour détecter une pression d'au moins un côté parmi le côté principal et le côté secondaire de la membrane de purification du sang (30) ; et
une unité de commande (13) qui ajuste un débit de la pompe de remplacement de fluide (170) de façon à ce qu'une pression sur la membrane de purification du sang (30) soit au sein d'une plage prédéterminée, sur la base d'un résultat de détection par le capteur de pression (51).

6. Dispositif de purification du sang (1) selon l'une quelconque des revendications 1 à 5, comprenant en outre une unité de commande (13) qui ajuste un débit de la pompe à sang (40) de façon à ce que le débit de la pompe à sang (40) soit inférieur à un débit de la pompe de remplacement de fluide (170) dans le processus d'amorçage.

7. Dispositif de purification du sang (1) selon l'une quelconque des revendications 1 ou 2, dans lequel
le circuit d'approvisionnement en dialysat (90) comporte un premier canal (112) équipé d'une pluralité de filtres (110, 111), et un second canal (113) équipé d'un ou plusieurs filtres (110, 111) avec un nombre inférieur au nombre de filtres du premier canal (112), et est configuré pour pouvoir basculer entre le premier canal (112) et le second canal (113).

8. Dispositif de purification du sang (1) selon la revendication 7, comprenant en outre une unité de commutation qui réalise le basculement entre le premier canal (112) et le second canal (113) de façon telle qu'en cas de réalisation d'un processus de rétrofiltration dans lequel une partie du dialysat ou tout le dialysat amené au côté secondaire de la membrane de purification du sang (30) de l'unité de purification du sang (20) est amené vers le côté primaire à travers la membrane de purification du sang (30), le dialysat est amené vers l'unité de purification du sang (20) à travers le premier canal (112), alors qu'en cas de non-réalisation du processus de rétrofiltration, le dialysat est amené vers l'unité de purification du sang (20) à travers le second canal (113).

9. Dispositif de purification du sang (1) selon la revendication 7 ou 8, comprenant en outre
un circuit de remplacement du fluide (150) connectant un côté aval de la pluralité de filtres (110, 111) dans le premier canal (112) au circuit sanguin (10), où
le circuit d'approvisionnement en dialysat (90) est configuré pour alimenter en dialysat le circuit de remplacement du fluide (150) à travers le premier canal (112).

10. Dispositif de purification du sang (1) selon la revendication 9, comprenant en outre
un circuit de connexion (151) connectant le circuit de remplacement du fluide (150) au circuit de renvoi du dialysat (92), où
le dispositif de purification du sang (1) est configuré pour pouvoir basculer entre la connexion au premier canal (112) du circuit de remplacement du fluide (150) et la connexion au circuit de renvoi du dialysat (92).

11. Dispositif de purification du sang (1) selon l'une quelconque des revendications 7 à 10, dans lequel le premier canal (112) et le second canal (113) partagent un ou plusieurs des filtres (110, 111).

12. Dispositif de purification du sang (1) selon la revendication 11, dans lequel
le ou les filtres (110, 111) partagés par les premier et second canaux (112, 113) sont disposés en amont des autres filtres.

13. Dispositif de purification du sang (1) selon l'une quelconque des revendications 7 à 12, dans lequel
dans un cas dans lequel le débit du dialysat amené à un filtre le plus en amont est inférieur à 700 ml/min, le dialysat est distribué au premier canal (112) alors que dans un cas dans lequel le débit est supérieur ou égal à 700 ml/min, le dialysat est distribué au second canal (113).

14. Procédé d'amorçage du dispositif de purification du sang (1) tel que défini dans la revendication 1, le procédé comprenant
une étape consistant à, en connectant une partie de collecte du sang (21) et une partie de retour du sang (23) d'un circuit sanguin (10) l'une à l'autre pour former une forme de boucle du circuit sanguin (10), alimenter en dialysat un côté secondaire d'une membrane de purification du sang (30) d'une unité de purification du sang (20) à travers des filtres situés dans un circuit d'approvisionnement en dialysat (90), provoquant l'écoulement du dialysat vers un côté principal via la membrane de purification du sang (30), et à renvoyer le dialysat du circuit sanguin (10)comportant le côté primaire vers un circuit de renvoi du dialysat (92), le nombre de filtres (110, 111) à travers lesquels passe le dialysat dans le circuit d'approvisionnement en dialysat (90) de l'étape étant plus grand que le nombre de filtres à travers lesquels le dialysat passe dans le circuit d'approvisionnement en dialysat (90) dans un procédé de purification du sang.
